# EUROPEAN PATENT APPLICATION

(11) **EP 3 037 535 A1**
(43) Date of publication of application: **29.06.2016**
(21) Application number: 14838742.6
(22) Date of filing: 20.08.2014
(51) Int. Cl.: C12N 15/09, C12Q 1/68, G01N 33/53

(54) **METHOD FOR MEASURING MODIFIED NUCLEIC-ACID BASE USING ABSORBENT POLYNUCLEOTIDE AND KIT THEREFOR**

(30) Priority: 21.08.2013 JP 2013171665
(71) Applicant: Fujirebio Inc., Shinjuku-ku Tokyo 163-0410 (JP)
(72) Inventor: MATSUNO, Tatsuki, Tokyo 163-0410 (JP); HORIIKE, Mariko, Tokyo 163-0410 (JP)
(74) Representative: Hoffmann Eitle
(86) International application number: PCT/JP2014/071705
(87) International publication number: WO 2015/025864

(57) **Abstract**

An object of the present invention is to provide a technique that suppresses a background value of a detection signal to construct an immunoassay system for detecting a modified nucleobase. Specifically, the present invention provides a method for measuring a modified nucleobase including incubating a nucleic acid sample and a capture probe in a solution and measuring a modified nucleobase using an antibody against the modified nucleobase in the presence of an absorbent polynucleotide in the obtained solution. The present invention also provides a kit for measuring a modified nucleobase including a capture probe, an absorbent polynucleotide, and an antibody against a modified nucleobase.

## Description

### TECHNICAL FIELD

The present invention relates to a method and a kit for measuring a modified nucleobase.

### BACKGROUND ART

Many techniques that detect nucleobases to which substances such as biotin are artificially introduced by immunoassays using antibodies in nucleic acid (e.g., DNA, RNA) detection have been reported. Techniques that detect naturally occurring modified nucleobases (e.g., methylcytosine and hydroxymethylcytosine) by immunoassays are also reported (Patent Literature 1, and Non Patent Literature 1 and 2).

### Prior Art Reference

### Patent Literature

Patent Literature 1: Japanese Patent Application Laid-open No. 2012-230019

### Non-Patent Literature

Non Patent Literature 1: Proll et al., DNA Research, 13, 37-42 (2006)
Non Patent Literature 2: Kurita et al., Anal. Chem., 2012, 84, 7533-7538

### SUMMARY OF INVENTION

### PROBLEM TO BE SOLVED BY THE INVENTION

The inventors of the present invention have found out that in constructing the above immunoassay system for detecting modified nucleobases, there is a problem in that a background value of a detection signal increases owing to non-specific binding of an antibody against a modified nucleobase to a nucleic acid (e.g., parts other than the modified nucleobase in a target nucleic acid containing the modified nucleobase and/or a capture probe). Consequently, a technique for suppressing the background value has been required to be developed in order to construct a high-sensitivity immunoassay system.

### MEANS FOR SOLVING PROBLEM

As a result of intensive investigations, the inventors of the present invention have succeeded in suppressing the background value and the like by using an absorbent polynucleotide in the measurement of a modified nucleobase in a target nucleic acid using an antibody against the modified nucleobase and have achieved the present invention.

That is, the present invention is as follows.
[1] A method for measuring a modified nucleobase
   comprising:
   (1) incubating a nucleic acid sample and a capture probe in a solution; and
   (2) measuring the modified nucleobase using an antibody against the modified nucleobase in the presence of an absorbent polynucleotide in the solution obtained at (1).
[2] The method according to [1], wherein
   the nucleic acid sample contains a target nucleic acid containing a modified nucleobase, and the steps (1) and (2) are performed by (1') and (2'), respectively:
   (1') reacting the nucleic acid sample containing the target nucleic acid containing the modified nucleobase and the capture probe in the solution by incubation to form a hybrid composed of the target nucleic acid and the capture probe; and
   (2') measuring the modified nucleobase using the antibody in the presence of the absorbent polynucleotide in the solution containing the hybrid.
[3] The method according to [1] or [2], wherein the target nucleic acid is a target nucleic acid potentially containing two or more modified nucleobases.
[4] The method according to any one of [1] to [3], further comprising adding the capture probe to a solution containing the nucleic acid sample to prepare the solution containing both the nucleic acid sample and the capture probe.
[5] The method according to any one of [1] to [4], wherein the nucleic acid sample is a sample containing a target DNA containing a modified nucleobase.
[6] The method according to any one of [1] to [5], wherein the absorbent polynucleotide contains 12 or more nucleotide residues.
[7] The method according to any one of [1] to [6], wherein the absorbent polynucleotide is a homopolynucleotide.
[8]The method according to any one of [1] to [7], wherein the absorbent polynucleotide contains a nucleotide residue containing cytosine, and the modified nucleobase is a modified cytosine.
[9] The method according to [8], wherein the modified cytosine is methylcytosine.
[10] The method according to any one of [2] to [9], wherein the capture probe is designed such that an unpaired part of the modified nucleobase is formed in a double-stranded structure part of the hybrid.
[11] The method according to any one of [2] to [10], wherein the capture probe is designed such that the modified nucleobase is present in a single-stranded structure part of the hybrid.
[12] The method according to any one of [1] to [11], wherein the measurement of the modified nucleobase using the absorbent polynucleotide and the antibody is performed by ELISA.
[13] A kit for measuring a modified nucleobase, the kit comprising:
   (I) a capture probe;
   (II) an absorbent polynucleotide; and
   (III) an antibody against the modified nucleobase.

### EFFECT OF INVENTION

The method and the kit of the present invention can measure a modified nucleobase in a target nucleic acid with high sensitivity by reducing a background value of a detection signal.

### BRIEF DESCRIPTION OF DRAWINGS

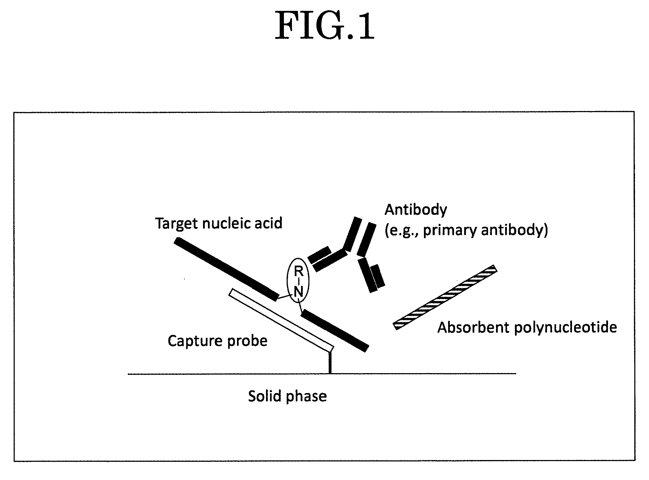

FIG. 1 is a diagram of an example of a measurement summary of a modified nucleobase in a target nucleic acid by the method of the present invention. R-N: a nucleotide residue having a modified nucleobase; N: a nucleotide residue having a nucleobase; and R: a substituent that a nucleobase has.
FIG. 2 is a diagram of signal values (background values) measured in measurement on the conditions of a target nucleic acid containing a modified nucleobase (-), a capture probe (+), an absorbent polynucleotide (+), and an antibody against the modified nucleobase.
FIG. 3 is a diagram of signal-to-noise ratios (S/N) calculated in the measurement of the modified nucleobase of each of the concentrations using a nucleic acid probe. In S/N, S indicates a signal value measured in measurement on the conditions of the target nucleic acid (+), the capture probe (+), the absorbent polynucleotide (+), and the antibody against the modified nucleobase (+), whereas N indicates a signal value (a background value) measured in measurement on the conditions of the target nucleic acid (-), the capture probe (+), and the antibody against the modified nucleobase (+).
FIG. 4 is a diagram of a comparison of an effect among Absorbent Polynucleotide 1 (poly C), Comparative Substance 1 (poly T), Comparative Substance 2 (poly A), and Comparative Substance 3 (poly G) in the measurement of the background value.
FIG. 5 is a diagram of a comparison of an effect among Absorbent Polynucleotide 1 (poly C), Comparative Substance 1 (poly T), Comparative Substance 2 (poly A), and Comparative Substance 3 (poly G) in the measurement of the S/N value.
FIG. 6 is a diagram of a comparison of an effect among Comparative Substance 4 (dCTP), Comparative Substance 5 (dATP), and Comparative Substance 6 (dGTP) in the measurement of the background value.
FIG. 7 is a diagram of a comparison of an effect among Comparative Substance 4 (dCTP), Comparative Substance 5 (dATP), and Comparative Substance 6 (dGTP) in the measurement of the S/N value.

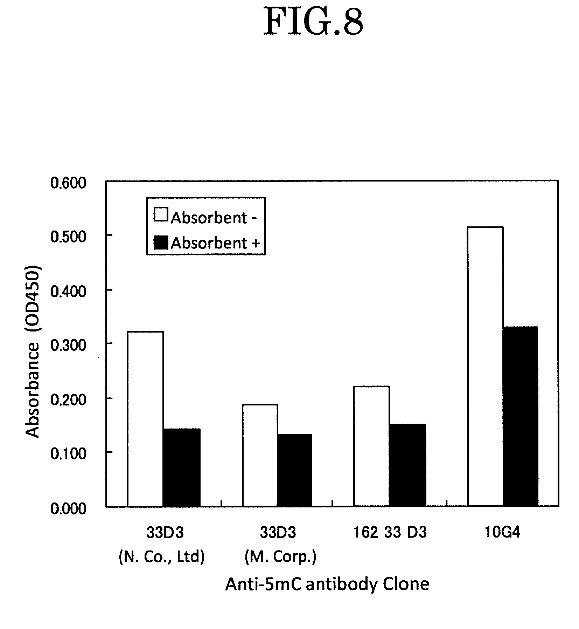

FIG. 8 is a diagram of background values measured in measurement on the conditions of the target nucleic acid containing the modified nucleobase (-), the capture probe (+), the absorbent polynucleotide (- or +), and various types of antibodies against the modified nucleobase (+). The used antibody clones are as follows: Clone33D3-N: an anti-methylcytosine antibody (manufactured by Nippon Gene Co., Ltd.); Clone33D3-M: an anti-methylcytosine antibody (manufactured by Millipore Corporation); Clone162 33 D3: an anti-methylcytosine antibody (manufactured by Millipore Corporation); and Clone10G4: an anti-methylcytosine antibody (manufactured by Zymo Research Corporation).
FIG. 9 is a diagram of an effect of the length of the absorbent polynucleotide (composed of one type of nucleotide residues) on suppression of the background value.
FIG. 10 is a diagram of an effect of the length of the absorbent polynucleotide (composed of one type of nucleotide residues) on increase in the S/N value.
FIG. 11 is a diagram of an effect of a nucleotide composition of the absorbent polynucleotide on suppression of the background value and increase in the S/N value. Numbers below the table indicate numbers of the absorbent polynucleotide.
FIG. 12 is a diagram of an effect of the nucleotide composition of the absorbent polynucleotide on suppression of the background value and increase in the S/N value. Numbers below the table indicate numbers of the absorbent polynucleotide.
FIG. 13 is a diagram of signal values (background values) measured in measurement on the conditions of the target nucleic acid containing the modified nucleobase (-), the capture probe (+), and the absorbent polynucleotide and the antibody against the modified nucleobase (+).
FIG. 14 is a diagram of signal-to-noise ratios (S/N) calculated in the measurement of the modified nucleobase of each of the concentrations using the nucleic acid probe. The used capture probe, absorbent polynucleotide, and antibody are similar to those in FIG. 13.
FIG. 15 is a diagram of signal values (background values) measured in measurement on the conditions of the target nucleic acid containing the modified nucleobase (-), the capture probe (+), and the absorbent polynucleotide and the antibody against the modified nucleobase (+).
FIG. 16 is a diagram of signal-to-noise ratios (S/N) calculated in the measurement of the modified nucleobase of each of the concentrations using the nucleic acid probe. The used capture probe, absorbent polynucleotide, and antibody are similar to those in FIG. 15.
FIG. 17 is a diagram of signal values (background values) measured in measurement on the conditions of the target nucleic acid containing the modified nucleobase (-), the capture probe (+), and the absorbent polynucleotide and the antibody against the modified nucleobase (+).
FIG. 18 is a diagram of signal-to-noise ratios (S/N) calculated in the measurement of the modified nucleobase of each of the concentrations using the nucleic acid probe. The used capture probe, absorbent polynucleotide, and antibody are similar to those in FIG. 17.
FIG. 19 is a diagram of signal values (background values) measured in measurement on the conditions of the target nucleic acid containing the modified nucleobase (-), the capture probe (+), and the absorbent polynucleotide and the antibody against the modified nucleobase (+).
FIG. 20 is a diagram of signal-to-noise ratios (S/N) calculated in the measurement of the modified nucleobase of each of the concentrations using the nucleic acid probe. The used capture probe, absorbent polynucleotide, and antibody are similar to those in FIG. 19.

### EMBODIMENTS FOR CARRYING OUT THE INVENTION

The present invention provides a method for measuring a modified nucleobase. The present invention includes:
(1) incubating a nucleic acid sample and a capture probe in a solution; and
(2) measuring the modified nucleobase using an antibody against the modified nucleobase in the presence of an absorbent polynucleotide in the solution obtained at (1).

The nucleic acid sample is a sample containing a target nucleic acid containing a modified nucleobase or a sample suspected to contain the target nucleic acid. The nucleic acid sample may also be an organism-derived biological sample, an environmental sample, or the like. Examples of the organism from which the biological sample is derived include animals such as mammals (e.g., humans, monkeys, mice, rats, rabbits, cattle, pigs, horses, goats, sheep) and birds (e.g., chickens), insects, microorganisms, plants, fungi, and fishes. The biological sample may also be a blood-related sample that is blood itself or a blood-derived sample (e.g., whole blood, blood serum, blood plasma), saliva, urine, milk, tissue or cell extract, or a combination thereof. The biological sample may further be derived from mammals contracting diseases (e.g., cancer, leukemia) or mammals that may contract diseases. Examples of the environmental sample include samples derived from soil, sea water, and fresh water that may contain nucleic acids. These samples may be subjected to another treatment before being used in the method of the present invention. Examples of the treatment include extraction and fragmentation (e.g., treatment with an enzyme such as a restriction enzyme) of nucleic acids (e.g., DNA such as genomic DNA, and RNA). Consequently, the method of the present invention may further include extracting a nucleic acid from the nucleic acid sample and/or fragmenting the nucleic acid. The method of the present invention may also further include treating the sample by centrifugation, extraction, filtration, precipitation, heating, freezing, refrigeration, stirring, or the like.

The target nucleic acid is DNA or RNA, and DNA is preferable. The target nucleic acid is also a coding region or a non-coding region (e.g., a transcriptional regulation region) of DNA. The number of nucleotide residues composing the target nucleic acid (that is, the length of the target nucleic acid) is not limited to a particular number so long as it enables hybridization with the capture probe and may be 10 or more, preferably 15 or more, and more preferably 20 or more, for example. The number of nucleotides composing the target nucleic acid is also not limited to a particular number and may be any number that may occur by fragmentation of genomic DNA, for example. The number of the nucleotides composing the target nucleic acid may be 10,000 or less, 5,000 or less, 2,000 or less, 1,000 or less, 500 or less, 200 or less, or 100 or less, for example. A GC content of the target nucleic acid is not limited to a particular value and may be 10% or more, 20% or more, 30% or more, 40% or more, 50 % or more, or 60% or more, for example. The GC content of the target nucleic acid is also 90% or less, 80% or less, or 70% or less, for example. The number of the modified nucleic acid that the target nucleic acid contains is not limited to a particular number so long as it is one or more (e.g., 1 to 100, 1 to 20, 1 to 10, or 1 to 5).

In the present invention, the modified nucleic acid refers to a nucleobase having a structure in which a normal nucleobase selected from the group consisting of adenine (A), guanine (G), cytosine (C), thymine (T), and uracil (U) is modified. When the target nucleic acid is DNA, examples of the term "nucleobase" in the expression "modified nucleobase" include adenine (A), guanine (G), cytosine (C), and thymine (T). When the target nucleic acid is RNA, examples thereof include adenine (A), guanine (G), cytosine (C), and uracil (U). The nucleobase is preferably cytosine (C). Examples of modification include introduction of a substituent to the normal nucleobase, elimination of a group (e.g., an amino group, an oxo group, a methyl group) that the normal nucleobase has, and exchange of a group that the normal nucleobase has with a substituent. The substituent is not limited to a particular type so long as it is one that naturally occurring nucleobases can have, and examples thereof include the substituents that the modified nucleobases in the modified nucleotides described in Administrative Instructions under the Patent Cooperation Treaty (the version enforced on January 1, 2009), Annex C, Appendix 2, Table 2: List of Modified Nucleotides have. The modified nucleotides described in the literature can be the same as the modified nucleotides described in "Guidelines for Preparation of Specifications Containing Nucleotide Sequences or Amino Acid Sequences (July of 2002) or (December of 2009)," Annex 2, Table 2: Modified Base Table disclosed by the Japan Patent Office. Consequently, concerning the modified nucleobase, the guidelines can also be referred to. The substituent is preferably methyl, hydroxymethyl, or carboxy and more preferably methyl or hydroxymethyl. The position of the modification such as substitution is not limited to a particular position and is the 2-position or the 4- to 6-positions, for example, and preferably the 5-position for the nucleobase (C, T, or U) having a pyrimidine ring and is the 2-position, the 6-position, or the 8-position, for example, for the nucleobase (A or G) having a purine ring.

The modified nucleobase is not limited to a particular type so long as it can naturally occur, and examples thereof include the modified nucleobases that the modified nucleotides described in Administrative Instructions under the Patent Cooperation Treaty (the version enforced on January 1, 2009), Annex C, Appendix 2, Table 2: List of Modified Nucleotides have. The modified nucleotides described in the literature can be the same as the modified nucleotides described in the guidelines, Annex 2, Table 2: Modified Base Table. Consequently, concerning the modified nucleobase, the guidelines can also be referred to. The modified nucleobase is preferably methylcytosine (e.g., 5-methylcytosine), hydroxymethylcytosine (e.g., 5-hydroxymethylcytosine), or carboxylcytosine (e.g., 5-carboxylcytosine). The modified nucleobase is more preferably methylcytosine (e.g., 5-methylcytosine) or hydroxymethylcytosine (e.g., 5-hydroxymethylcytosine). It is known that the modified nucleobase brings changes in functions of nucleic acids (e.g., a change in the transcriptional regulation capability of a certain gene).

The capture probe is a nucleic acid molecule that can hybridize with the target nucleic acid. The capture probe is composed of nucleic acids homogeneous and/or heterogeneous to the target nucleic acid. The term "homogeneous" means that the capture probe has the same backbone structure as a backbone structure (a structure composed of a sugar moiety and a phosphoric acid moiety) of the target nucleic acid as the whole of the backbone structure. The term "heterogeneous" means that the capture probe has a backbone structure different from the backbone structure (the structure composed of the sugar moiety and the phosphoric acid moiety) of the target nucleic acid as part or the whole of the backbone structure. Consequently, the type of the capture probe is determined in accordance with the type of the target nucleic acid. When the target nucleic acid is DNA, for example, a DNA probe can be used as the capture probe of the homogeneous nucleic acid, and a nucleic acid probe other than the DNA probe can be used as the capture probe of the heterogeneous nucleic acid. When the target nucleic acid is natural RNA, a normal RNA probe composed of RNA homogeneous with the natural RNA can be used as the capture probe of the homogeneous nucleic acid, and a nucleic acid probe other than the normal RNA probe can be used as the capture probe of the heterogeneous nucleic acid.

Examples of the capture probe include DNA probes, RNA probes, peptide nucleic acid (PNA) probes, locked nucleic acid (also called LNA or bridged nucleic acid (BNA)) probes, phosphorothioate (S-) nucleic acid probes, and chimera nucleic acid probes in which two or more such nucleic acid probes are coupled and/or mixed with each other (the chimera nucleic acid probe inevitably contains a nucleic acid heterogeneous to the target nucleic acid). Examples of the RNA probes include a normal RNA probe composed of a natural ribonucleotide having a hydroxy group at the 2'-position and a modified RNA probe composed of a ribonucleotide the 2'-position hydroxy group of which is modified. The modified RNA probe may be a ribonuclease-resistant RNA probe. Examples of the modified RNA probe include a 2'-O-alkylated RNA probe. The 2'-O-alkylated RNA probe is preferably 2'-O-C₁₋₆ alkylated RNA probe. The C₁₋₆ alkyl group of the C₁₋₆ alkylation is a linear, branched, or cyclic C₁₋₆ alkyl group, and examples thereof include a methyl group, an ethyl group, a propyl group (e.g., n-propyl ,iso-propyl), a butyl group (e.g., n-butyl, isobutyl, sec-butyl, tert-butyl), a pentyl group, and a hexyl group. In terms of easiness of manufacture and acquisition or the like, the 2'-O-C₁₋₆ alkylated RNA probe is preferably a 2'-O-methylated RNA probe.

The number of nucleotide residues composing the capture probe (that is, the length of the capture probe) is not limited to a particular number so long as it is long enough to be able to hybridize with the target nucleic acid and may be 10 or more, preferably 15 or more, and more preferably 20 or more, for example. The number of nucleotides composing the capture probe may also be 100 or less, 80 or less, 60 or less, 50 or less, 40 or less, or 30 or less, for example. A GC content of the target nucleic acid is not limited to a particular value and may be 10% or more, 20% or more, 30% or more, 40% or more, 50% or more, or 60% or more, for example. The GC content of the target nucleic acid may also be 90% or less, 80% or less, or 70% or less, for example. The capture probe can be prepared by a method of synthesizing a probe known in the relevant field, for example.

The capture probe is used in the form of being free or the form of being immobilized to the solid phase (described below). Consequently, the capture probe may be labeled with a substance or group that enables immobilization to the solid phase. The labeling is performed either at the 5'-end or the 3'-end, for example. Examples of the substance or group that enables immobilization to the solid phase include substances or groups that enable covalent binding to the solid phase and affinity substances. Examples of the substances or groups that enable covalent binding to the solid phase include a thiol group or substances having a thiol group (the thiol group introduced into the capture probe can bind to a maleimide group on the solid phase) and an amino group or substances having an amino group (the amino group introduced into the capture probe can bind to maleic anhydride on the solid phase). Examples of the affinity substances include streptavidin, biotin, digoxigenin, dinitrophenol, fluorescein, and fluorescein isothiocyanate. In this case, the solid phase coated with another affinity substance having affinity with the affinity substance that the capture probe has can be used.

At the step (1), the incubation is performed in an appropriate solution on the condition that, when the target nucleic acid is contained in the nucleic acid sample, a hybridization reaction of the capture probe (being free or immobilized to the solid phase described below) and the target nucleic acid in the nucleic acid sample is made possible. As the solution, a buffer solution containing a salt (e.g., sodium citrate) and other components (e.g., a surfactant) can be used, for example. A hybridization temperature is 15°C to 95°C, for example (preferably 25°C to 65°C). The incubation may be performed in the absence or presence of the absorbent polynucleotide (being free or immobilized to the solid phase described below). In other words, the solution provided for the incubation may contain the absorbent polynucleotide described below in addition to the nucleic acid sample and the capture probe or does not necessarily contain the absorbent polynucleotide. This is because the absorbent polynucleotide does not hybridize with the target nucleic acid and the capture probe. Consequently, when the solution provided for the incubation contains the nucleic acid sample, the capture probe, and the absorbent polynucleotide, the order of addition of the nucleic acid sample, the capture probe, and the absorbent polynucleotide to the solution is not limited to particular order as a matter of course. When the solution provided for the incubation does not contain the absorbent polynucleotide, the absorbent polynucleotide is added to the solution after the incubation. The addition of the absorbent polynucleotide to the solution may be performed before or after addition of an antibody against the modified nucleobase to the solution or may be performed at the same time therewith.

When the nucleic acid sample does not contain the target nucleic acid, even when the nucleic acid sample and the capture probe are incubated in the solution, a hybrid is not formed. In this case, the modified nucleobase cannot be detected at the step (2) described below, but it can be determined that the modified nucleobase is not present in the nucleic acid sample.

When the nucleic acid sample contains the target nucleic acid not containing the modified nucleobase (in other words, the target nucleic acid containing non-modified nucleobases alone), by incubating the nucleic acid sample and the capture probe in the solution, the target nucleic acid not containing the modified nucleobase and the capture probe react with each other, whereby a hybrid composed of the target nucleic acid and the capture probe is formed. In this case, the modified nucleobase cannot be detected at the step (2) described below, but it can be determined that the modified nucleobase is not present in the nucleic acid sample (even though the target nucleic acid is present) or, in other words, that a certain nucleobase in the target nucleic acid is not modified.

When the nucleic acid sample contains the target nucleic acid containing the modified nucleobase, by incubating the nucleic acid sample and the capture probe in the solution, the target nucleic acid containing the modified nucleobase and the capture probe react with each other, whereby a hybrid composed of the target nucleic acid and the capture probe is formed. In this case, it can be determined that the modified nucleobase is present at the step (2) described below, and the modified nucleobase can also be quantified.

In the present invention, the hybrid is a hybridization complex having a double-stranded structure of the target nucleic acid and the capture probe formed through hybridization. Examples of the structure of the hybrid are as illustrated in the following Table 1. The double-stranded structure may be formed at the entire region of the target nucleic acid and the capture probe as illustrated in (a). The double-stranded structure may also be formed at a partial region of the target nucleic acid or the capture probe as illustrated in (b) to (i). The hybrid may have a single-stranded structure of the target nucleic acid either at a 5'-end region or a 3'-end region [e.g., (b) to (e)] or may also have a single-stranded structure of the target nucleic acid both at the 5'-end region and the 3'-end region [e.g., (i)] in addition to the double-stranded structure, for example. The hybrid may also have a single-stranded structure of the capture probe either at the 5'-end region or the 3'-end region [e.g., (b), (c), (f), (g)] or may also have a single-stranded structure of the target nucleic acid both at the 5'-end region and the 3'-end region [e.g., (h)] in addition to the double-stranded structure. The single-stranded structure is a structure formed by either one or both of the target nucleic acid and the capture probe, which have formed the hybrid, having a non-hybridized region of one or more nucleotide residues at the 5'-end and/or the 3'-end. (a) to (i) present structures in which at least two end regions among the 5'-end region and the 3'-end region of the target nucleic acid and the 5'-end region and the 3'-end region of the capture probe hybridize. However, in the hybrid, these two end regions are also not necessarily required to hybridize; both the 3'-end region of the target nucleic acid and the 5'-end region of the capture probe may have single-stranded structure parts (that is, non-hybridized regions), and both the 5'-end region of the target nucleic acid and the 3'-end region of the capture probe may have single-stranded structure parts.

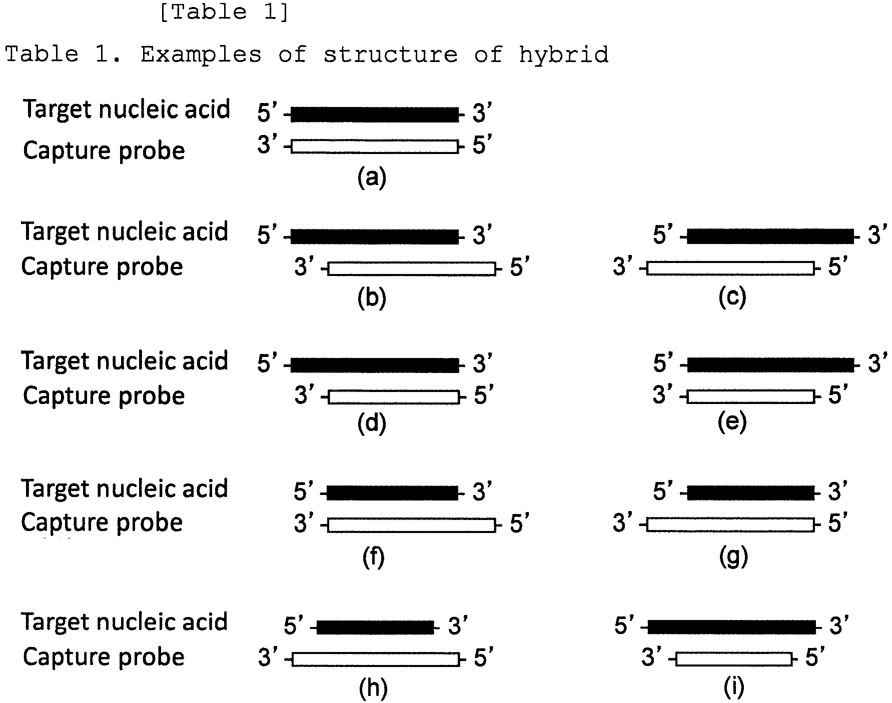

In the hybrid, the number of nucleotide residues of the target nucleic acid and the capture probe corresponding to the double-stranded structure part (that is, the length of the double-stranded structure part) is not limited to a particular number so long as it is long enough to enable hybridization with the target nucleic acid and may be 10 or more, preferably 15 or more, and more preferably 20 or more, for example. The number of the nucleotide residues of the target nucleic acid and the capture probe corresponding to the double-stranded structure part may also be 100 or less, 80 or less, 60 or less, or 50 or less, 40 or less, or 30 or less, for example. A GC content in the double-stranded structure part is not limited to a particular value and may be 10% or more, 20% or more, 30% or more, 40% or more, 50% or more, or 60% or more, for example. The GC content in the double-stranded structure part may also be 90% or less, 80% or less, or 70% or less, for example.

In the hybrid, the number of nucleotide residues of the target nucleic acid and the capture probe corresponding to the single-stranded structure parts at its 5'-end region or 3'-end region (that is, the length of each of the single-stranded structure parts) is not limited to a particular number so long as it is one or more and is two or more, three or more, four or more, five or more, six or more, seven or more, eight or more, nine or more, 10 or more, 15 or more, 20 or more, or 50 or more, for example. The number is not limited to a particular number and may also be 10,000 or less, 5,000 or less, 2,000 or less, 1,000 or less, 500 or less, 200 or less, or 100 or less, for example. The capture probe may be designed such that the single-stranded structure part is formed at the 5'-end region or the 3'-end region in the hybrid.

In an embodiment, the capture probe may be designed such that an unpaired part of the modified nucleobase will be formed in the double-stranded structure part of the hybrid. The unpaired part of the modified nucleobase can be introduced to facilitate detection of the modified nucleobase by an antibody. To form the unpaired part, a capture probe having a nucleotide sequence that is not perfectly complementary to the target nucleic acid in the double-stranded structure part may be used, for example.

An example of the capture probe in which the unpaired part of the modified nucleobase is formed in the double-stranded structure part of the hybrid is a capture probe lacking a nucleotide residue complementary to a nucleotide residue having a modified nucleobase in the target nucleic acid [e.g., (I) in Table 2]. The capture probe may be a capture probe lacking one nucleotide residue alone complementary to the nucleotide residue having the modified nucleobase in the target nucleic acid [e.g., (I-1) in Table 2] or a capture probe lacking two or more (2 to 20, 2 to 10, or 2 to 5, for example) adjacent nucleotide residues containing the nucleotide residue having the modified nucleobase in the target nucleic acid [e.g., (I-2) in Table 2]. The number of the nucleotide residue having the modified nucleobase in the unpaired part is not limited to a particular number so long as it is one or more as described above. Concerning the nucleic acid probe, refer to Patent Literature 1 and Non Patent Literature 2, for example. When the position of the nucleotide residue having the modified nucleobase in the target nucleic acid to be measured is determined, such design is made possible.

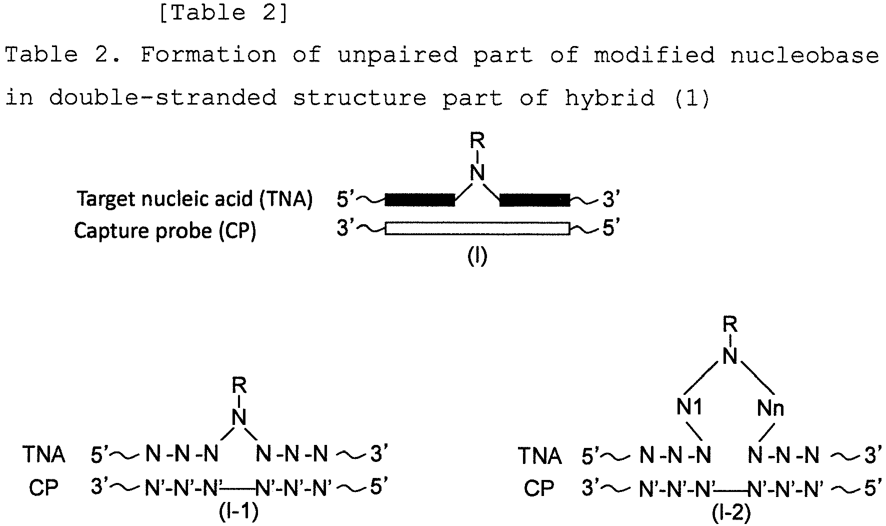

R-N: Nucleotide residue having modified nucleobase
N:Nucleotide residue having non-modified nucleobase composing target nucleic acid
N':Nucleotide residue composing capture probe
n in Nn: Number of nucleotide residues in bulge (loop) part (e.g., 2 to 20. When n is 2, either N1 or N2 or both N1 and N2 may have substituent R.)
R: Substituent that nucleobase has

Another example of the capture probe in which the unpaired part of the modified nucleobase is formed in the double-stranded structure part of the hybrid is a capture probe having a nucleotide residue noncomplementary to the nucleotide residue having the modified nucleobase in the target nucleic acid [e.g., (I') in Table 2]. The capture probe may be a capture probe having one nucleotide residue alone noncomplementary with respect to the nucleotide residue having the modified nucleobase in the target nucleic acid [e.g., (I'-1) in Table 2] or a capture probe in which two or more (2 to 20, 2 to 10, or 2 to 5, for example) adjacent nucleotide residues containing the nucleotide residue having the modified nucleobase in the target nucleic acid are noncomplementary [e.g., (I'-2) in Table 2]. When the position of the nucleotide residue having the modified nucleobase in the target nucleic acid to be measured is determined, such design is made possible.

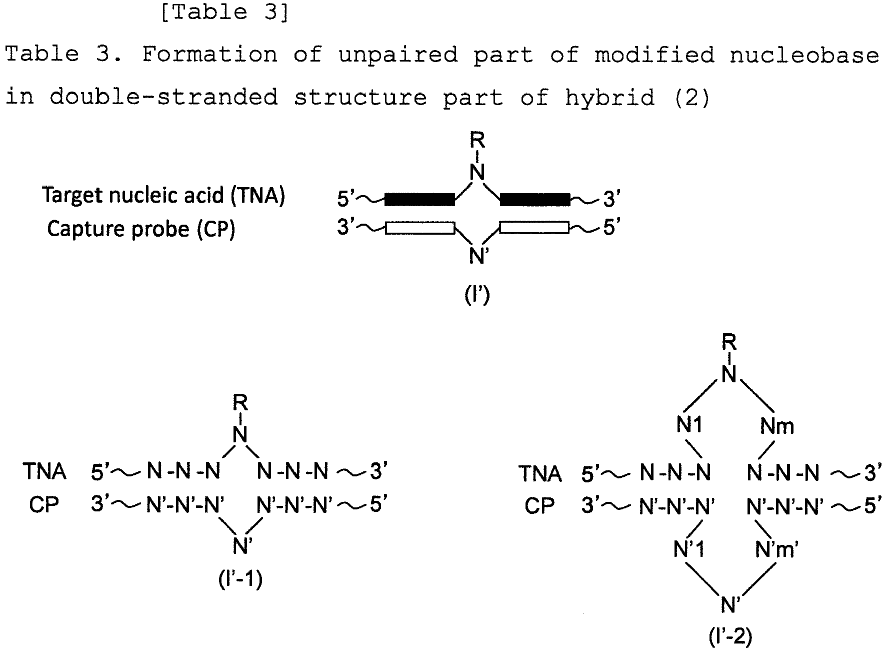

R-N: Nucleotide residue having modified nucleobase
N: Nucleotide residue having non-modified nucleobase composing target nucleic acid
N': Nucleotide residue composing capture probe
m in Nm: Number of nucleotide residues in noncomplementary part (N1 to Nm) (e.g., 2 to 20. When m is 2, either N1 or N2 or both N1 and N2 may have substituent R.)
m' in N'm': Number of nucleotide residues in noncomplementary part (N'1 to N'm') (e.g., 2 to 20) R: Substituent that nucleobase has

In another embodiment, the capture probe may be designed such that the modified nucleobase is present in the single-stranded structure part of the hybrid. In the single-stranded structure part (the target nucleic acid) of the hybrid, the modified nucleobase may be present at a non-end part of the single-stranded structure part on the 5'-end side [e.g., (II) in Table 4], may be present at an end part of the single-stranded structure part on the 5'-end side [e.g., (III) in Table 4], may be present at a non-end part of the single-stranded structure part on the 3'-end side [e.g., (IV) in Table 4], may be present at an end part of the single-stranded structure part on the 3'-end side [e.g., (V) in Table 4], or may be any combination of two, three, or four of these. The capture probe may be designed such that the single-stranded structure part (the target nucleic acid) of the hybrid contains the modified nucleobase in such a manner. The number of the nucleotide residue having the modified nucleobase in the single-stranded structure part is not limited to a particular number so long as it is one or more as described above. Alternatively, the capture probe may be designed such that the modified nucleobase is present in the single-stranded structure part of the hybrid and that the unpaired part of the modified nucleobase will further be formed in the double-stranded structure part of the hybrid as described above. When the position of the nucleotide residue having the modified nucleobase in the target nucleic acid to be measured is determined, such design is made possible.

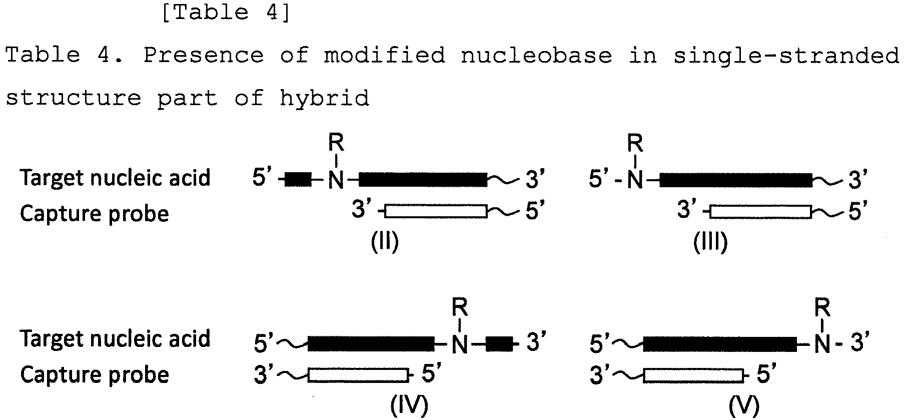

R-N: Nucleotide residue having modified nucleobase
N: Nucleotide residue having non-modified nucleobase
R: Substituent that nucleobase has

The method of the present invention may further include adding the capture probe to a solution containing the nucleic acid sample to prepare the solution containing both the nucleic acid sample and the capture probe. The capture probe can be added to the nucleic acid sample in the form of a solid or as a solution.

When the solution for incubation is prepared from the nucleic acid sample containing the target nucleic acid containing the modified nucleobase, the concentration of the target nucleic acid in the solution is not limited to a particular value so long as it is detectable by the method of the present invention and may be 0.01 nM or more, preferably 0.1 nM or more, more preferably 1 nM or more, further more preferably 5 nM or more, and particularly preferably 10 nM or more, for example. The concentration of the target nucleic acid in the solution may also be 1 M or less, 100 mM or less, 10 mM or less, 1 mM or less, 100 µM or less, 10 µM or less, or 1 µM or less, for example. Since the concentration of the target nucleic acid in the nucleic acid sample is unknown in many cases, and it may be difficult to strictly set a concentration of the target nucleic acid. Depending on the type of the nucleic acid sample, the concentration of the target nucleic acid that can be contained in the nucleic acid sample can empirically be predicted to some extent, or the concentration of the target nucleic acid is determined (in a case when although the size and/or concentration of the target nucleic acid is separately measured, the presence or absence of the modified nucleobase in the target nucleic acid and the content of the modified nucleobase in the target nucleic acid are unknown, for example). In such cases, setting of the concentration of the target nucleic acid may be attempted as described above.

The concentration of the capture probe in the solution is not limited to a particular value so long as the target nucleic acid is detectable by the method of the present invention and may be 0.01 nM or more, preferably 0.1 nM or more, more preferably 1 nM or more, further more preferably 5 nM or more, and particularly preferably 10 nM or more, for example. The concentration of the capture probe in the solution may also be 1 M or less, 100 mM or less, 10 mM or less, 1 mM or less, 100 µM or less, 10 µM or less, or 1 µM or less, for example. Consequently, the capture probe may be added to the solution such that such a concentration will be achieved.

In a preferable embodiment, the target nucleic acid may be a target nucleic acid potentially containing two or more modified nucleobases. The number of the modified nucleobases potentially contained in the target nucleic acid is not limited to a particular number so long as it is two or more and is 2 to 30, 2 to 20, 2 to 10, or 2 to 5 (e.g., 2, 3, 4, or 5), for example. When a plurality of modified nucleobases are contained in the target nucleic acid, it has been revealed that even when the concentration of the target nucleic acid in the solution used for the hybridization of the target nucleic acid and a capture probe is extremely low (e.g., 0.1 nM or more), the modified nucleobases can be measured with high sensitivity. Consequently, the method of the present invention can use a capture probe that is designed so as to hybridize with the target nucleic acid potentially containing two or more modified nucleobases. When the number of nucleobases potentially modified in the target nucleic acid to be measured is determined, such design is made possible.

The modified nucleobase is measured using an antibody against the modified nucleobase in the presence of the absorbent polynucleotide in a solution containing the hybrid. In the measurement, although the solution obtained at the step (1) may be used as it is, addition of another solution and/or replacement of the solution with another solution may be performed in order to perform measurement in a solution more suitable for the measurement of the modified nucleobase by the antibody. The replacement can be performed by adding the solution obtained at the step (1) to a solid phase, immobilizing the hybrid that can be contained in the solution to the solid phase, removing the solution from the solid phase, washing the solid phase with a cleaning liquid as needed, and adding another solution (e.g., a solution containing the absorbent polynucleotide and/or the antibody against the modified nucleobase) thereto, for example. The solution used in the measurement is not limited to a particular type so long as it is a solution suitable for an antigen-antibody reaction.

The measurement can be performed by immunological methodology. Examples of the immunological methodology include an enzyme immunoassay (EIA) (e.g., direct competitive ELISA, indirect competitive ELISA, and sandwich ELISA), a radioimmunoassay (RIA), a fluoroimmunoassay (FIA), immunochromatography, a luminescence immunoassay, a spin immunoassay, Western blot, and latex agglutination.

In the present invention, the absorbent polynucleotide means a polynucleotide containing a nucleotide residue containing a nucleobase (that is, a non-modified nucleobase) composing the modified nucleobase as an object to be measured of the present invention (hereinafter, such a nucleotide residue may be referred to as an "effective nucleotide residue"). In other words, the "modified nucleobase" as a target of the antibody used in the present invention differs from the "nucleobase" contained in the effective nucleotide residue in the presence or absence of modification. When the target nucleic acid is DNA and the modified nucleobase is a modified adenine, a modified guanine, a modified cytosine, or a modified thymine, for example, the absorbent polynucleotide is a polynucleotide containing a polynucleotide residue containing adenine, guanine, cytosine, or thymine, respectively, as a nucleobase. When the target nucleic acid is RNA and the modified nucleobase is a modified adenine, a modified guanine, a modified cytosine, or a modified uracil, the absorbent polynucleotide is a polynucleotide containing a polynucleotide residue containing adenine, guanine, cytosine, or uracil, respectively, as a nucleobase. The modified cytosine is preferable as the modified nucleobase, and thus the absorbent polynucleotide is preferably a polynucleotide containing a nucleotide residue containing cytosine.

The absorbent polynucleotide can be composed of a nucleic acid homogeneous or heterogeneous to the target nucleic and preferably is composed of a nucleic acid homogeneous with respect to the target nucleic acid. The term "homogeneous" means that the absorbent polynucleotide has the same backbone structure as a backbone structure (a structure composed of a sugar moiety and a phosphoric acid moiety) of the target nucleic acid. The term "heterogeneous" means that the absorbent polynucleotide has a backbone structure different from the backbone structure (the structure composed of the sugar moiety and the phosphoric acid moiety) of the target nucleic acid. Consequently, the type of the absorbent polynucleotide may be determined in accordance with the type of the target nucleic acid. When the target nucleic acid is DNA, for example, although an absorbent polynucleotide of the homogeneous nucleic acid (DNA) or an absorbent polynucleotide of the heterogeneous nucleic acid (RNA) can be used, the absorbent polynucleotide of the homogeneous nucleic acid (DNA) is preferably used. When the target nucleic acid is natural RNA, although an absorbent polynucleotide of the homogeneous nucleic acid (RNA) or an absorbent polynucleotide of the heterogeneous nucleic acid (DNA) can be used, the absorbent polynucleotide of the homogeneous nucleic acid (RNA) is preferably used. DNA is preferable as the target nucleic acid, and thus the absorbent polynucleotide is preferably DNA. The modified cytosine is preferable as the modified nucleobase, and thus the absorbent polynucleotide is preferably DNA containing a nucleotide residue containing cytosine.

The total number of nucleotide residues composing the absorbent polynucleotide (that is, the length of the absorbent polynucleotide) may be 12 or more, preferably 14 or more, 16 or more, 18 or more, or 20 or more, for example. The total number of nucleotides composing the absorbent polynucleotide may also be 100 or less, 80 or less, 70 or less, or 60 or less, for example. The number of effective nucleotide residues in the absorbent polynucleotide is not limited to a particular number so long as suppression of the background value and/or increase in the S/N ratio is revealed in the present invention and may be 12 or more, preferably 14 or more, 16 or more or 18 or more, and more preferably 20 or more, for example. The number of the effective nucleotide residues in the absorbent polynucleotide may also be 100 or less, 80 or less, 70 or less, 60 or less, 50 or less, 40 or less, or 30 or less, for example. The absorbent polynucleotide can be prepared by a method for polymerizing nucleotides known in the relevant field, for example.

The absorbent polynucleotide is used in the form of being free or the form of being immobilized to the solid phase (described below). Consequently, the absorbent polynucleotide may be labeled with a substance or group that enables immobilization to the solid phase. The labeling is performed either at the 5'-end or the 3'-end, for example. Examples of the substance or group that enables immobilization to the solid phase include the substances or groups described above for the capture probe.

In one embodiment, the absorbent polynucleotide is a homopolynucleotide. The homopolynucleotide refers to a polynucleotide composed of homogeneous nucleotide residues alone. When the target nucleic acid is DNA and the modified nucleobase is a modified adenine, a modified guanine, a modified cytosine, or a modified thymine, for example, the homopolynucleotide is DNA composed of nucleotide residues alone containing adenine, guanine, cytosine, or thymine, respectively, as a nucleobase. When the target nucleic acid is RNA and the modified nucleobase is a modified adenine, a modified guanine, a modified cytosine, or a modified uracil, the absorbent polynucleotide is RNA composed of nucleotide residues alone containing adenine, guanine, cytosine, or uracil, respectively, as a nucleobase. The absorbent polynucleotide is preferably a polynucleotide composed of nucleotide residues alone containing cytosine and more preferably DNA composed of nucleotide residues alone containing cytosine.

In another embodiment, the absorbent polynucleotide is a heteropolynucleotide. The heteropolynucleotide refers to a polynucleotide composed of two or more heterogeneous nucleotide residues. When the target nucleic acid is DNA and the modified nucleobase is a modified adenine, a modified guanine, a modified cytosine, or a modified thymine, for example, the absorbent polynucleotide each is a polynucleotide containing the effective nucleotide residue and one or more nucleotide residues other than the effective nucleotide residue. When the target nucleic acid is RNA and the modified nucleobase is a modified adenine, a modified guanine, a modified cytosine, or a modified uracil, the absorbent polynucleotide each is a polynucleotide containing the effective nucleotide residue and one or more nucleotide residues other than the effective nucleotide residue. The modified cytosine is preferable as the modified nucleobase, and the absorbent polynucleotide is preferably a polynucleotide containing a nucleotide residue containing cytosine (the effective nucleotide residue) and one or more nucleotide residues other than the nucleotide residue and more preferably DNA containing a nucleotide residue containing cytosine (the effective nucleotide residue) and one or more nucleotide residues other than the nucleotide residue. When the absorbent polynucleotide is a heteropolynucleotide, a content of the effective nucleotide residue in the absorbent polynucleotide is not limited to a particular value so long as suppression of the background value and/or increase in the S/N value is revealed in the present invention and may be 10% or more, 20% or more, 30% or more, 40% or more, 50% or more, 60% or more, 70% or more, 80% or more, 90% or more, or 95% or more, for example.

The antibody against the modified nucleobase may be a polyclonal antibody or a monoclonal antibody. The antibody against the modified nucleobase may be any isotype of immunoglobulin (e.g., IgG, IgM, IgA, IgD, IgE, IgY). The antibody against the modified nucleobase may be a full-length antibody. The full-length antibody refers to an antibody containing a heavy chain and a light chain, each of the chains containing a variable region and a constant region, respectively (e.g., an antibody containing two Fab parts and an Fc part). The antibody against the modified nucleobase may also be an antibody fragment derived from the full-length antibody. The antibody fragment is part of the full-length antibody, and examples thereof include F(ab')₂, Fab', Fab, and Fv. The antibody against the modified nucleobase may also be a modified antibody such as a single-stranded antibody. The antibody against the modified nucleobase may further be an antibody used as a primary antibody in an immunoassay such as ELISA, and in this case, a secondary antibody is used in combination.

The antibody against the modified nucleobase may have affinity for the modified nucleobase, a nucleoside having the modified nucleobase (a structural unit composed of the modified nucleobase and 2'-deoxyribose or ribose), a nucleotide having the modified nucleobase (a structural unit composed of the modified nucleobase, 2'-deoxyribose or ribose, and phosphate), or two or more nucleotides containing the nucleotide having the modified nucleobase (e.g., an oligonucleotide composed of two to five nucleotides). Examples of the antibody against the modified nucleobase when the target nucleic acid is DNA include 1) antibodies against a deoxyribonucleoside having a modified nucleobase selected from the group consisting of 2'-deoxy-modified adenosine, 2'-deoxy-modified guanosine, 2'-deoxy-modified cytidine, and 2'-deoxy-modified thymidine, 2) antibodies against a deoxyribonucleotide having a modified nucleobase selected from the group consisting of 2'-deoxy-modified adenosine 5'-phosphate, 2'-deoxy-modified guanosine 5'-phosphate, 2'-deoxy-modified cytidine 5'-phosphate, and 2'-deoxy-modified thymidine 5'-phosphate, and 3) antibodies against two or more deoxyribonucleotides containing the above deoxyribonucleotide having the modified nucleobase. Examples of the antibody against the modified nucleobase when the target nucleic acid is RNA include 1') antibodies against a nucleoside having a modified nucleobase selected from the group consisting of modified adenosine, modified guanosine, modified cytidine, and modified uridine, 2') antibodies against a ribonucleotide having a modified nucleobase selected from the group consisting of modified adenosine 5'-phosphate, modified guanosine 5'-phosphate, modified cytidine 5'-phosphate, and modified uridine 5'-phosphate and 3') antibodies against two or more ribonucleotides containing the above ribonucleotide having the modified nucleobase.

For the antibody against the modified nucleobase, an antibody prepared by using a complex of the modified nucleobase, the nucleoside having the modified nucleobase, the nucleotide having the modified nucleobase, or the two or more nucleotides containing the nucleotide having the modified nucleobase and a carrier protein (e.g., BSA, KLH) as an antigen can be used, for example. Various antibodies against the modified nucleobase prepared using such complexes are commercially available, and the method of the present invention may use a commercially available antibody, for example. The method of the present invention may also use the antibody against the modified nucleobase prepared as follows, for example.

The polyclonal antibody against the modified nucleobase can be acquired by administering the complex as the antigen together with a commercially available adjuvant (e.g., a complete or incomplete Freund's adjuvant) to an animal subcutaneously or intra-abdominally about two to four times every 2 to 3 weeks, collecting whole blood about 3 to about 10 days after the final immunity, and purifying an antiserum, for example. Examples of the animal to which the antigen is administered include mammals such as rats, mice, rabbits, goats, cattle, guinea pigs, and hamsters.

The monoclonal antibody against the modified nucleobase can be prepared by cell fusion, for example. The complex is administered together with a commercially available adjuvant to a mouse subcutaneously or intra-abdominally two to four times, collecting the spleen or a lymph node about three days after the final administration, and collecting white blood cells, for example. These white blood cells and a myeloma cell (e.g., NS-1) are subjected to cell fusion to obtain a hybridoma producing a monoclonal antibody against the factor. Examples of the cell fusion include a PEG method and a voltage pulse method. The hybridoma producing a desired monoclonal antibody can be selected by detecting an antibody that specifically binds to an antigen using known EIA, RIA, or the like in cultivated supernatant. Cultivation of the hybridoma producing the monoclonal antibody can be performed in vitro or in vivo such as in a mouse, a rat, or preferably mouse ascites, and the antibody can be acquired from the cultivated supernatant of the hybridoma or animal ascites. The monoclonal antibody may be any isotype of IgG, IgM, IgA, IgE, and the like. Alternatively, in vitro methods such as a phage display method (Ulman et al, Proc. Natl. Acad. Sci. U.S.A., 90, 1184-89 (1993)) and an ADLib system (WO2004/011644) are also known as methods for preparing a monoclonal antibody, and such methods may be used to prepare the antibody against the modified nucleobase.

The antibody against the modified nucleobase may be used while being immobilized to a solid phase. Examples of the solid phase include supports such as particles (e.g., magnetic particles), membranes (e.g., a nitrocellulose membrane), glass, plastic, and metal, containers such as plates (e.g., a multiwell plate), and devices. The antibody may also be provided in the form of being impregnated into a medium such as filter paper. The antibody against the modified nucleobase may be labeled with a labeling substance. Examples of the labeling substance include enzymes (e.g., peroxidase, alkaline phosphatase, luciferase, β-galactosidase), affinity substances (e.g., streptavidin ,biotin), fluorescent substances or proteins (e.g., fluorescein, fluorescein isothiocyanate, rhodamine, green fluorescent protein, red fluorescent protein), luminescent substances (e.g., luciferin, aequorin), and radioactive substances (e.g., ³H, ¹⁴C, ³²P, ³⁵S, ¹²⁵I). When a secondary antibody is used in the method of the present invention, the secondary antibody may be labeled with such a labeling substance.

The measurement of the modified nucleobase using the absorbent polynucleotide and the antibody against the modified nucleobase is performed qualitatively or quantitatively, and the presence or absence or the amount of the modified nucleobase can be evaluated. In the present invention, the measurement of the modified nucleobase intends not only the measurement of the modified nucleobase itself but also the measurement of the target nucleic acid containing the modified nucleobase.

The measurement of the modified nucleobase using the absorbent polynucleotide and the antibody against the modified nucleobase can be achieved by causing the absorbent polynucleotide and the antibody against the modified nucleobase to coexist in the solution obtained at (1). The concentration of the absorbent polynucleotide in the solution is not limited to a particular value so long as suppression of the background value and/or increase in the S/N ratio is revealed in the present invention and may be 0.1 nM or more, preferably 1 nM or more, more preferably 2 nM or more, further more preferably 10 nM or more, and particularly preferably 50 nM or more, for example. The concentration of the capture probe in the solution may also be 1 M or less, 100 mM or less, 10 mM or less, 1 mM or less, 100 µM or less, or 10 µM or less, for example. Consequently, the absorbent polynucleotide may be added to the solution such that such a concentration will be achieved.

The measurement of the presence or absence of the modified nucleobase may be performed as follows, for example:
(2-1) in the solution obtained at the step (1), performing an assay using the absorbent polynucleotide and the antibody against the modified nucleobase to measure a signal value;
(2-2) in a solution that does not contain the target nucleic acid containing the modified nucleobase and contains the capture probe, performing an assay using the absorbent polynucleotide and the antibody against the modified nucleobase to measure a background value; and (2-3) comparing the signal value with the background value to evaluate the presence or absence of the modified nucleobase.

In the measurement of the modified nucleobase, the signal value and the background value are values (e.g., absorbance, the degree of fluorescence, the degree of coloration, and radioactivity) that are measured using a label binding to the antibody against the modified nucleobase or the secondary antibody (when the secondary antibody is used).

The measurement of the amount of the modified nucleobase may be performed together with the measurement of the background value, for example. Specifically, the measurement of the amount of the modified nucleobase may be performed as follows:
(2-1') in the solution obtained at the step (1), performing an assay using the absorbent polynucleotide and the antibody against the modified nucleobase to measure a signal value;
(2-2') in a solution that does not contain the target nucleic acid containing the modified nucleobase and contains the capture probe, performing an assay using the absorbent polynucleotide and the antibody against the modified nucleobase to measure a background value;
(2-3') correcting the signal value with the background value to obtain a corrected signal value; and
(2-4') based on the corrected signal value, evaluating the amount of the modified nucleobase.

Alternatively, the measurement of the amount of the modified nucleobase may be performed using a preparation. Specifically, the measurement of the amount of the modified nucleobase may be performed as follows:
(2-1") in the solution obtained at the step (1), performing an assay using the absorbent polynucleotide and the antibody against the modified nucleobase to measure a signal value;
(2-2") in a solution containing the target nucleic acid containing the modified nucleobase (preparation) and the capture probe, performing an assay using the absorbent polynucleotide and the antibody against the modified nucleobase to measure a value for calibration; and
(2-3") comparing the signal value with the value for calibration to evaluate the amount of the modified nucleobase.

The measurement using the preparation may be performed in combination with the measurement of the background value.

In a specific embodiment, the method of the present invention may be performed by ELISA. When the nucleic acid sample contains the target nucleic acid containing the modified nucleobase, for example, the method of the present invention by ELISA may be performed as follows:
(i) incubating the nucleic acid sample containing the target nucleic acid containing the modified nucleobase and a capture probe labeled with a first affinity substance in a solution to form a hybrid composed of the target nucleic acid and the capture probe;
(ii) immobilizing the hybrid to a solid phase treated with a second affinity substance;
(iii) reacting a primary antibody against the modified nucleobase with the hybrid immobilized to the solid phase in the presence of the absorbent polynucleotide to obtain a primary complex of the primary antibody and the hybrid;
(iv) reacting a secondary antibody labeled with a labeling substance with the primary complex to obtain a secondary complex of the secondary antibody and the primary antibody; and
(v) using the labeling substance that the secondary antibody in the secondary complex has, measuring the presence and/or the amount of the formed hybrid (in other words, the modified nucleobase).

The first affinity substance and the second affinity substance are used in a combination having mutual affinity (e.g., a combination of biotin and streptavidin). The method of the present invention may include (i') incubating the nucleic acid sample containing the target nucleic acid containing the modified nucleobase and the capture probe immobilized to a solid phase in a solution to form a hybrid composed of the target nucleic acid and the capture probe in place of the steps (i) and (ii). In this case, obtaining the capture probe immobilized to the solid phase (e.g., adding the capture probe labeled with the first affinity substance to the solid phase treated with the second affinity substance) may further be included. The method of the present invention may also include washing the solid phase before the step (iii). The secondary antibody may be an antibody that recognizes the primary antibody alone (e.g., an antibody that binds to the constant region of the primary antibody) and may also be an antibody that recognizes both the primary antibody in the secondary complex and the primary complex. In addition, the method of the present invention including (i) to (v) can be performed in accordance with the methodology described in detail in the specification.

The present invention also provides a kit for measuring a modified nucleobase. The kit of the present invention includes:
(I) a capture probe;
(II) an absorbent polynucleotide; and
(III) an antibody against the modified nucleobase.

The capture probe, the absorbent polynucleotide, and the antibody against a modified nucleobase are as described above. The capture probe may be labeled with the affinity substance, and the antibody against a modified nucleobase may be labeled with the labeling substance, for example. The kit of the present invention may further contain the components as described above including the above ones such as the affinity substance, the labeling substance, the secondary antibody, a detection reagent for the secondary antibody (e.g., when the secondary antibody is labeled with an enzyme, a substrate for the enzyme), and the solid phase. The solid phase may be treated with the affinity substance. The kit of the present invention may also contain the preparation of the modified nucleobase or the preparation of the target nucleic acid containing the modified nucleobase as solution or as powder.

The kit of the present invention contains the components in the form of being isolated from each other or in the form of being mixed with each other. In the kit of the present invention, the components may be provided in the form of being contained in different containers (e.g., a tube, a plate), for example, and the absorbent polynucleotide and the antibody against a modified nucleobase may be provided in the form of being mixed with each other (e.g., in the same solution), for example. Alternatively, the kit of the present invention may be provided in the form of a device. Specifically, all the components may be provided in the form of being contained in a device. Alternatively, part of the components may be provided in the form of being contained in a device, whereas the rest may be provided in the form of not being contained in the device (e.g., the form of being contained in a different container). In this case, the components not contained in the device may be used by being injected into the device in the measurement of a target substance.

### EXAMPLES

Although the following describes the present invention in more detail with reference to examples, the present invention is not limited to these examples.

### Example 1: Use of Absorbent Polynucleotide in Measurement of Modified Nucleobase

The nucleotide sequence of the target nucleic acid (DNA) is 5'-TTGCGCGGCGTC[C]GTCCTGTTGACTTC-3' (SEQ ID NO: 1, [C] is 5-methylcytosine), and the nucleotide sequence of the capture probe for capturing the target nucleic acid is 5'-GAAGTCAACAGGACGACGCCGCGCAA-3' (SEQ ID NO: 2); those artificially synthesized by Hokkaido System Science Co., Ltd. were used. The capture probe was designed such that, when a hybrid with the target nucleic acid was formed, an unpaired part was formed at the nucleobase [C] in the double-stranded structure part. The nucleotide sequence of the absorbent polynucleotide (DNA) containing cytosine is 5'-CCCCCCCCCCCCCCCCCCCC-3' (SEQ ID NO: 3, Absorbent Polynucleotide 1); that artificially synthesized by Hokkaido System Science Co., Ltd. was used.

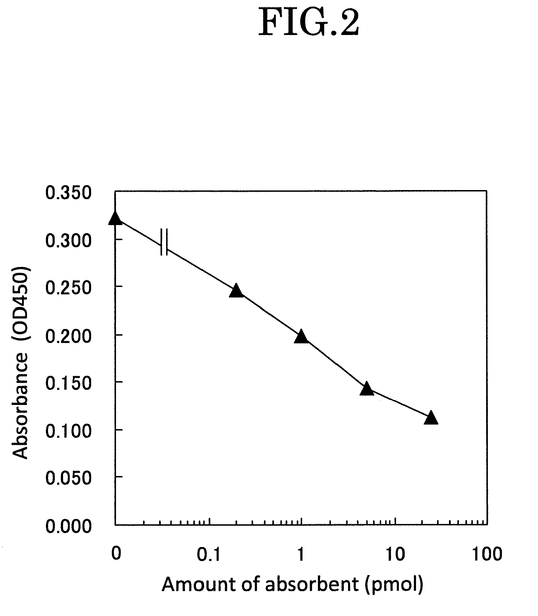

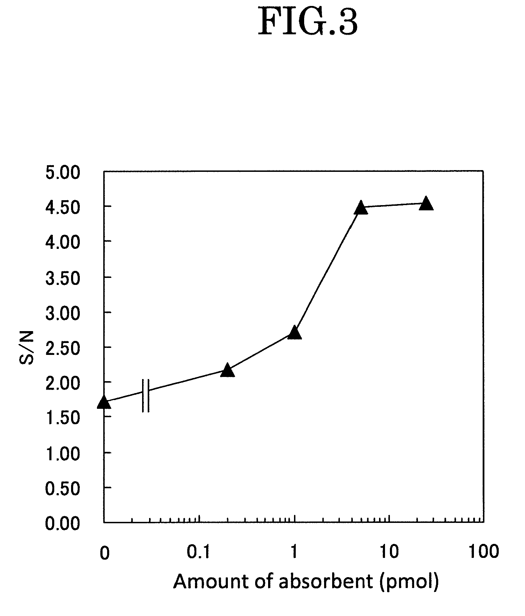

The measurement of the modified nucleobase using the absorbent polynucleotide was carried out as follows. First, the target nucleic acid containing 5-methylcytosine (1 pmol) and the capture probe (5 pmol) were dissolved in 100 µL of a hybridization buffer (4x SSC, 0.3% Tween20) (in a nucleic acid sample solution, the concentration of the target nucleic acid was 10 nM, and the concentration of the capture probe was 50 nM) and were reacted at 60°C for 2 hours to form a hybrid of the target nucleic acid and the capture probe. A solution not containing the target nucleic acid was also prepared, and a similar operation was performed. The solution after the hybridization reaction in an amount of 100 µL was added to a streptavidin-coated plate (manufactured by Thermo Scientific) and was reacted at 37°C for 30 minutes to immobilize the hybrid on the streptavidin plate. The streptavidin plate was washed twice with 300 µL of PBS-T, and a solution of 75 ng/mL of an anti-methylcytosine antibody (Clone33D3 manufactured by Nippon Gene Co., Ltd.) containing 0.2, 1, 5, or 25 pmol of Absorbent Polynucleotide 1 was added thereto by 100 µL each and was reacted at 37°C for 1 hour (in the solution, the concentration of Absorbent Polynucleotide 1 was 2, 10, 50, or 250 nM, respectively). At the same time, also for a solution of 75 ng/mL of the anti-methylcytosine antibody (Clone33D3 manufactured by Nippon Gene Co., Ltd.) not containing Absorbent Polynucleotide 1, a similar operation was performed. The streptavidin plate was washed three times with 300 µL of PBS-T, and 250 ng/mL of a peroxidase-labeled anti-IgG antibody (manufactured by Thermo Scientific) was added thereto by 100 µL each and was reacted at 37°C for 30 minutes. After the streptavidin plate was washed three times with 300 µL of PBS-T, 3,3',5,5'-tetramethylbenzidine was added thereto by 100 µL each and was reacted in a dark place at room temperature for 15 minutes. Thereafter, a 2N hydrochloric acid solution was added thereto by 100 µL each, and absorbance at 450 nm was measured by a microplate reader (Arvo manufactured by PerkinElmer, Inc.). The results are listed and illustrated in Table 4 and FIGs. 2 and 3.

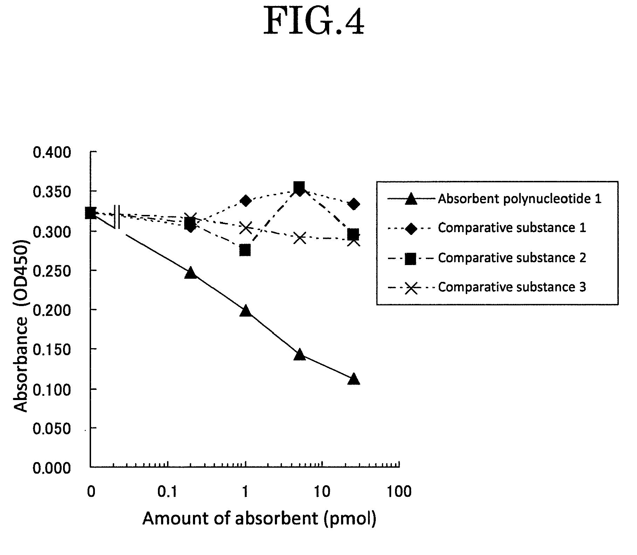

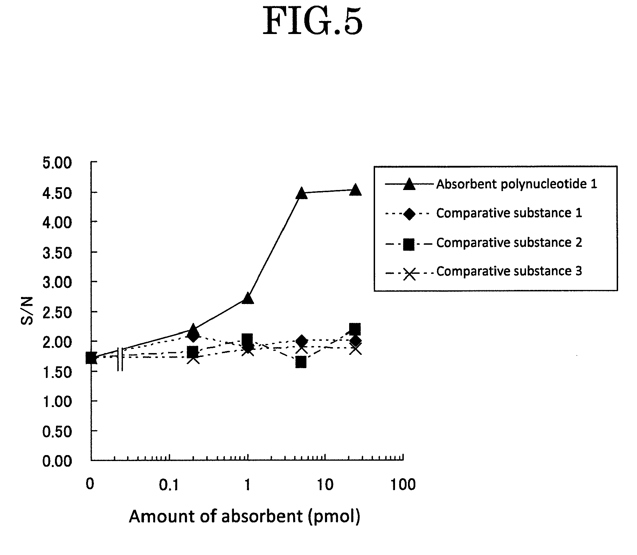

Tests were carried out by a method similar to that in Example 1 except that polynucleotides (DNA) not containing cytosine were used in place of Absorbent Polynucleotide 1. The sequences of the used polynucleotides (DNA) not containing cytosine are 5'-TTTTTTTTTTTTTTTTTTTT-3' (SEQ ID NO: 4, Comparative Substance 1), 5'-AAAAAAAAAAAAAAAAAAAA-3' (SEQ ID NO: 5, Comparative Substance 2), and 5'-GGGGGGGGGGGGGGGGGGGG-3' (SEQ ID NO: 6, Comparative Substance 3). The results are listed and illustrated in Table 4 and FIGs. 4 and 5.

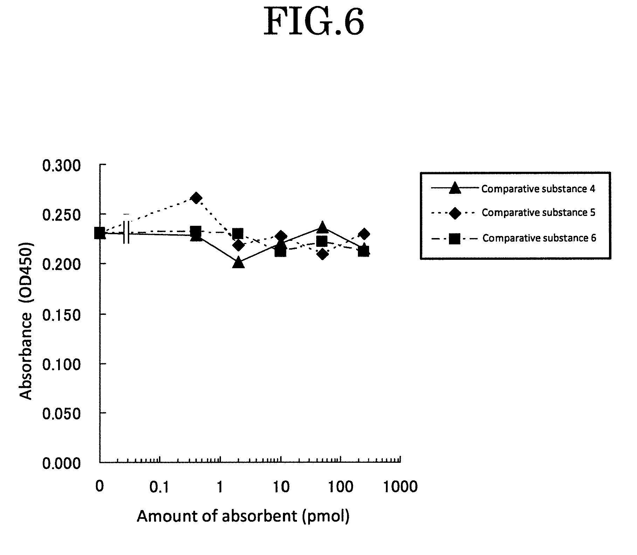

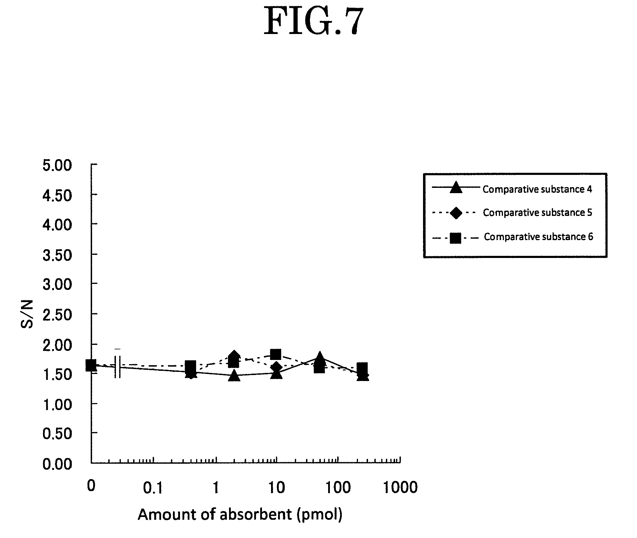

Furthermore, tests were carried out by a method similar to that in Example 1 except that deoxyribonucleotide triphosphates were used in place of Absorbent Polynucleotide 1. The used deoxyribonucleotide triphosphates are deoxycytidine triphosphate (dCTP) (Comparative Substance 4), deoxyadenosine triphosphate (dATP) (Comparative Substance 5), and deoxyguanosine triphosphate (dGTP) (Comparative Substance 6). dCTP, dATP, and dGTP were each added in 0.4, 2, 10, 50, or 250 pmol. The results are listed and illustrated in Table 5 and FIGs. 6 and 7.

As a result of the tests, no effect was revealed except for Absorbent Polynucleotide 1 as the polynucleotide containing cytosine (Table 5 and FIGs. 4 and 5). The nucleotides revealed no effect (Table 6 and FIGs. 6 and 7). These facts indicate that in the measurement of the modified nucleobase using the antibody against the modified nucleobase (e.g., a modified cytosine), the absorbent polynucleotide (e.g., a polynucleotide containing a nucleotide residue containing a non-modified nucleobase such as cytosine) is useful for suppressing the background value of the detection signal and increase a signal-to-noise ratio (an S/N value).

**[Table 5]**

| Table 5. Effect of absorbent polynucleotide on suppression of detection signal - comparison with other polynucleotides | | | | |
|---|---|---|---|---|
| Absorbent | Amount of absorbent | Target nucleic acid (DNA) | OD450 | S/N |
| Absorbent polynucleotide 1 (SEQ ID NO:3) | 0.2pmol | 1pmol | 0.538 | 2.18 |
| | | 0mol | 0.246 * | |
| | 1pmol | 1pmol | 0.539 | 2.71 |
| | | 0mol | 0.199 * | |
| | 5pmol | 1pmol | 0.639 | 4.48 |
| | | 0mol | 0.143 * | |
| | 25pmol | 1pmol | 0.510 | 4.54 |
| | | 0mol | 0.112 * | |
| Comparative substance 1 (SEQ ID NO:4) | 0.2pmol | 1pmol | 0.633 | 2.08 |
| | | 0mol | 0.305 * | |
| | 1pmol | 1pmol | 0.639 | 1.89 |
| | | 0mol | 0.338 * | |
| | 5pmol | 1pmol | 0.701 | 2.00 |
| | | 0mol | 0.350 * | |
| | 25pmol | 1pmol | 0.665 | 2.00 |
| | | 0mol | 0.333 * | |
| Comparative substance 2 (SEQ ID NO:5) | 0.2pmol | 1pmol | 0.560 | 1.81 |
| | | 0mol | 0.310 * | |
| | 1pmol | 1pmol | 0.556 | 2.02 |
| | | 0mol | 0.276 * | |
| | 5pmol | 1pmol | 0.584 | 1.65 |
| | | 0mol | 0.355 * | |
| | 25pmol | 1pmol | 0.646 | 2.19 |
| | | 0mol | 0.296 * | |
| Comparative substance 3 (SEQ ID NO:6) | 0.2pmol | 1pmol | 0.538 | 1.71 |
| | | 0mol | 0.315 * | |
| | 1pmol | 1pmol | 0.562 | 1.85 |
| | | 0mol | 0.303 * | |
| | 5pmol | 1pmol | 0.547 | 1.88 |
| | | 0mol | 0.290 * | |
| | 25pmol | 1pmol | 0.540 | 1.88 |
| | | 0mol | 0.288 * | |
| - | | 1pmol | 0.552 | 1.71 |
| | | 0mol | 0.323 * | |

| | | | | |
|---|---|---|---|---|
| * Background value | | | | |

**[Table 6]**

| Table 6. Effect of nucleotide on suppression of detection signal | | | | |
|---|---|---|---|---|
| Absorbent | Amount of absorbent | Target nucleic acid (DNA) | OD450 | S/N |
| Comparative substance 4 (dCTP) | 0.4pmol | 1pmol | 0.337 | 1.47 |
| | | 0mol | 0.229 * | |
| | 2pmol | 1pmol | 0.354 | 1.76 |
| | | 0mol | 0.201 * | |
| | 10pmol | 1pmol | 0.331 | 1.50 |
| | | 0mol | 0.221 * | |
| | 50pmol | 1pmol | 0.349 | 1.47 |
| | | 0mol | 0.237 * | |
| | 250pmol | 1pmol | 0.328 | 1.52 |
| | | 0mol | 0.216 * | |
| Comparative substance 5 (dATP) | 0.4pmol | 1pmol | 0.391 | 1.47 |
| | | 0mol | 0.266 * | |
| | 2pmol | 1pmol | 0.360 | 1.65 |
| | | 0mol | 0.219 * | |
| | 10pmol | 1pmol | 0.364 | 1.60 |
| | | 0mol | 0.227 * | |
| | 50pmol | 1pmol | 0.375 | 1.79 |
| | | 0mol | 0.209 * | |
| | 250pmol | 1pmol | 0.345 | 1.50 |
| | | 0mol | 0.230 * | |
| Comparative substance 6 (dGTP) | 0.4pmol | 1pmol | 0.368 | 1.58 |
| | | 0mol | 0.232 * | |
| | 2pmol | 1pmol | 0.364 | 1.59 |
| | | 0mol | 0.230 * | |
| | 10pmol | 1pmol | 0.383 | 1.81 |
| | | 0mol | 0.211 * | |
| | 50pmol | 1pmol | 0.371 | 1.68 |
| | | 0mol | 0.222 * | |
| | 250pmol | 1pmol | 0.343 | 1.62 |
| | | 0mol | 0.212 * | |
| Absorbent polynucleotide 1 (SEQ ID NO:3) | 5pmol | 1pmol | 0.378 | 2.86 |
| | | 0mol | 0.132 * | |
| - | | 1pmol | 0.376 | 1.63 |
| | | 0mol | 0.231 * | |

| | | | | |
|---|---|---|---|---|
| * Background value | | | | |

From the foregoing, it has been revealed that use of the absorbent polynucleotide enables a high-sensitivity immunoassay of the modified nucleobase.

### Example 2: Investigation of Difference in Antibody Clone

Test were carried out by a method similar to that in Example 1 except that 100 ng/mL of an anti-methylcytosine antibody (Clone33D3 manufactured by Millipore Corporation), 100 ng/mL of an anti-methylcytosine antibody (Clone162 33 D3 manufactured by Millipore Corporation), and 10 ng/mL of an anti-methylcytosine antibody (Clone10G4 manufactured by Zymo Research Corporation) were used in place of 50 ng/mL of the anti-methylcytosine antibody (Clone33D3 manufactured by Nippon Gene Co., Ltd.).

As a result of the tests, it was revealed that even when different antibody clones were used, an effect of suppressing the background value of the detection signal was achieved (Table 7 and FIG. 8).

**[Table 7]**

| Table 7. Effect of absorbent polynucleotide on suppression of detection signal - difference among antibodies | | | | | |
|---|---|---|---|---|---|
| Antibody Clone No. | Absorbent polynucleotide | Target nucleic acid (DNA) | OD450 | S/N | Remarks |
| 33D3 (Manufactured by Nippon Gene) | - | 1pmol | 0.552 | 1.71 | Antibody concentration 50ng/mL |
| | | 0mol | 0.323* | | |
| | + | 1pmol | 0.639 | 4.48 | |
| | | 0mol | 0.143* | | |
| 33D3 (Manufactured by Millipore) | - | 1pmol | 0.417 | 2.23 | Antibody concentration 100ng/mL |
| | | Omol | 0.187* | | |
| | + | 1pmol | 0.378 | 2.86 | |
| | | 0mol | 0.132* | | |
| 162 33 D3 | - | 1pmol | 0.526 | 2.37 | Antibody concentration 100ng/mL |
| | | 0mol | 0.221* | | |
| | + | 1pmol | 0.445 | 2.95 | |
| | | 0mol | 0.151* | | |
| 10G4 | - | 1pmol | 0.692 | 1.35 | Antibody concentration 10ng/mL |
| | | 0mol | 0.514* | | |
| | + | 1pmol | 0.659 | 2.00 | |
| | | 0mol | 0.329* | | |

| | | | | | |
|---|---|---|---|---|---|
| * Background value | | | | | |

From the foregoing, it has been revealed that the effect of the present invention is not limited to a specific antibody clone.

### Example 3: Investigation of Length of Absorbent Polynucleotide Containing Cytosine

Tests were performed by a method similar to that in Example 1 except that 5 pmol of Absorbent Polynucleotides 2 to 11 were added in place of Absorbent Polynucleotide 1. A summary of Absorbent Polynucleotides 2 to 11 is as listed in Table 8.

**[Table 8]**

| Table 8. Summary of Absorbent Polynucleotides 2 to 11 | | |
|---|---|---|
| Absorbent polynucleotide | Length | Sequence |
| 2 | 10 | 5'-CCCCCCCCC C-3'(SEQ ID NO:7) |
| 3 | 30 | 5'-CCCCCCCCCCCCCCCCCCCCCCCC-3' (SEQ ID NO:8) |
| 4 | 40 | 5' -CCCCCCCCCCCCCCCCCCCCCCCCCCCCCCCCCCCCC-3' (SEQ ID NO:9) |
| 5 | 50 | 5'-CCCCCCCCCCCCCCCCCCCCCCCCCCCCCCCCCCCCCCCCCCCCC- 3'(SEQ ID NO:10) |
| 6 | 60 | 5'-CCCCCCCCCCCCCCCCCCCCCCCCCCCCCCCCCCCCCCCCCCCC CCCCCCCCC-3'(SEQ ID NO:11) |
| 7 | 70 | 5'-CCCCCCCCCCCCCCCCCCCCCCCCCCCCCCCCCCCCCCCCCCCCCCCCCC CCCCCCCCCCCCCCCCCCCC-3'(SEQ ID NO:12) |
| 8 | 12 | 5'-CCCCCCCCCCCC-3'(SEQ ID NO:13) |
| 9 | 14 | 5'-CCCCCCCCCCCCCC-3'(SEQ ID NO:14) |
| 10 | 16 | 5'-CCCCCCCCCCCCCCCC-3'(SEQ ID NO:15) |
| 11 | 18 | 5'-CCCCCCCCCCCCCCCCCC-3'(SEQ ID NO:16) |

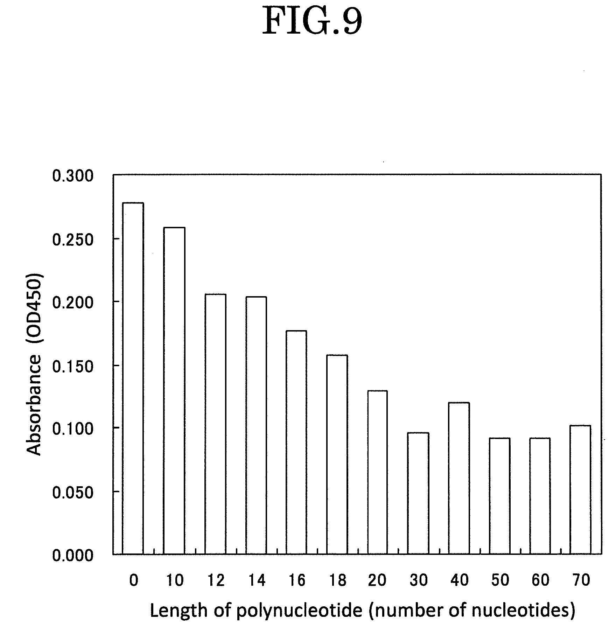

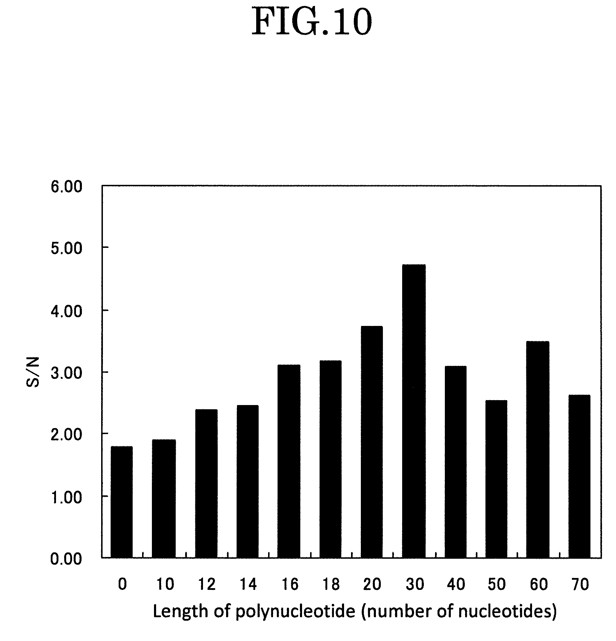

As a result of the tests, it was revealed that when the length of the absorbent polynucleotide containing cytosine was 12 or more, a remarkable effect was obtained (Table 9 and FIGs. 9 and 10).

**[Table 9]**

| Table 9. Background value and S/N value in measurement of modified nucleobase by absorbent polynucleotides of various lengths | | | | |
|---|---|---|---|---|
| Absorbent polynucleotide | Length | Target nucleic acid (DNA) | OD450 | S/N |
| - | - | 1pmol | 0.498 | 1.79 |
| | | 0mol | 0.278 * | |
| 1 | 20 | 1pmol | 0.482 | 3.72 |
| | | 0mol | 0.129 * | |
| 2 | 10 | 1pmol | 0.486 | 1.89 |
| | | 0mol | 0.258 * | |
| 3 | 30 | 1pmol | 0.452 | 4.72 |
| | | 0mol | 0.096 * | |
| 4 | 40 | 1pmol | 0.369 | 3.09 |
| | | 0mol | 0.119 * | |
| 5 | 50 | 1pmol | 0.233 | 2.54 |
| | | 0mol | 0.092 * | |
| 6 | 60 | 1pmol | 0.320 | 3.49 |
| | | 0mol | 0.091 * | |
| 7 | 70 | 1pmol | 0.267 | 2.63 |
| | | 0mol | 0.101 * | |
| 8 | 12 | 1pmol | 0.489 | 2.38 |
| | | 0mol | 0.206 * | |
| 9 | 14 | 1pmol | 0.496 | 2.44 |
| | | 0mol | 0.203 * | |
| 10 | 16 | 1pmol | 0.548 | 3.10 |
| | | 0mol | 0.177 * | |
| 11 | 18 | 1pmol | 0.499 | 3.18 |
| | | 0mol | 0.157 * | |

| | | | | |
|---|---|---|---|---|
| * Background value | | | | |

From the foregoing, it has been revealed that in the measurement of the modified nucleobase using the antibody against the modified nucleobase, use of the polynucleotide containing 12 or more non-modified nucleobases of one type alone as the absorbent polynucleotide produces an especially excellent effect.

### Example 4: Investigation of Content of Cytosine in Absorbent Polynucleotide Containing Cytosine

Tests were performed by a method similar to that in Example 1 except that 5 pmol of Absorbent Polynucleotides 12 to 23 were added in place of Absorbent Polynucleotide 1.

**[Table 10]**

| Table 10. Summary of Absorbent Polynucleotide 12 to 23 | | |
|---|---|---|
| Absorbent polynucleotide | Length | Sequence |
| 12 | 20 | 5'-AAAAAACAAAAAACAAAAAA-3' (SEQ ID NO:17) |
| 13 | 20 | 5'-AAACAAACAAACAAACAAAA-3' (SEQ ID NO:18) |
| 14 | 20 | 5'-AACAACAACAACAACAACAA- 3' (SEQ ID NO:19) |
| 15 | 20 | 5'-AACACACACAACACACACAA-3' (SEQ ID NO:20) |
| 16 | 20 | 5'-ACACACACACACACACACAC-3' (SEQ ID NO:21) |
| 17 | 20 | 5'-CCACACACACCACACACACC-3' (SEQ ID NO:22) |
| 18 | 20 | 5'-CCACCACCACCACCACCACC-3' (SEQ ID NO:23) |
| 19 | 20 | 5'-CCCACCCACCCACCCACCCC-3' (SEQ ID NO:24) |
| 20 | 20 | 5'-CCCCCCACCCCCCACCCCCC-3' (SEQ ID NO:25) |
| 21 | 30 | 5'-CCACACACACCACACACACAAAAAAAAAAC-3' (SEQ ID NO:26) |
| 22 | 40 | 5'-CCACACACACCACACACACAAAAAAAAAAAAAAAAAAAA C-3' (SEQ ID NO:27) |
| 23 | 50 | 5'-CCACACACACCACACACACAAAAAAAAAAAAAAAAAAAAAAAAAAAAAAAAC- 3'(SEQ ID NO:28) |

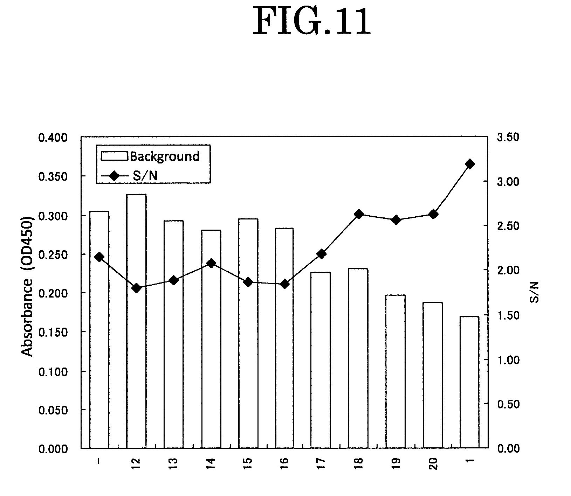

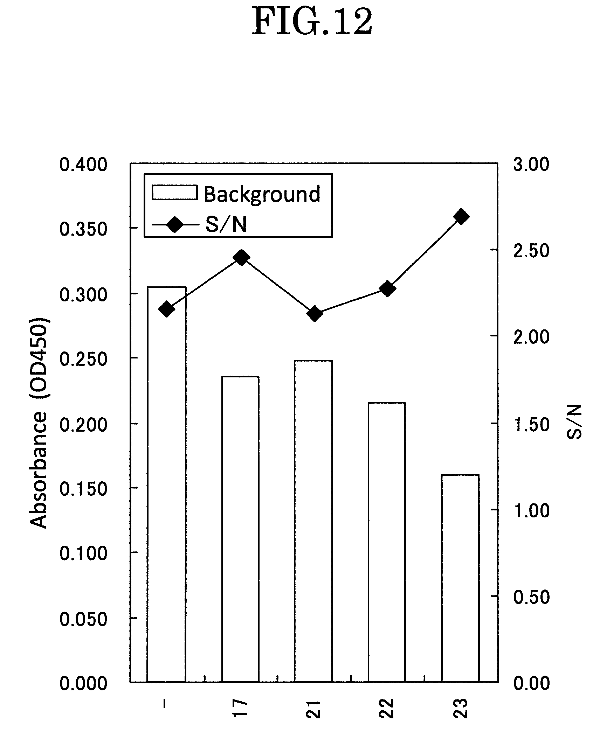

When the absorbent polynucleotide contained 12 or more cytosines, a remarkable effect was revealed (Table 11 and FIG. 11). Also in the polynucleotides having different lengths, an effect was revealed when 12 or more cytosines were contained; the importance of the number of cytosines, not the content thereof, was revealed (Table 12 and FIG. 12).

**[Table 11]**

| Table 11. Background value and S/N value in measurement of modified nucleobase by absorbent polynucleotides of various lengths | | | | | |
|---|---|---|---|---|---|
| Absorbent polynucleotide | Length | Number of cytosines | Target nucleic acid (DNA) | OD450 | S/N |
| - | - | - | 1pmol | 0.656 | 2.16 |
| | | | 0mol | 0.304* | |
| 1 | 20 | 20 | 1pmol | 0.540 | 3.19 |
| | | | 0mol | 0.169* | |
| 12 | 20 | 2 | 1pmol | 0.588 | 1.80 |
| | | | 0mol | 0.326* | |
| 13 | 20 | 4 | 1pmol | 0.551 | 1.88 |
| | | | 0mol | 0.293* | |
| 14 | 20 | 6 | 1pmol | 0.581 | 2.07 |
| | | | 0mol | 0.280* | |
| 15 | 20 | 8 | 1pmol | 0.549 | 1.86 |
| | | | 0mol | 0.294* | |
| 16 | 20 | 10 | 1pmol | 0.522 | 1.85 |
| | | | 0mol | 0.282* | |
| 17 | 20 | 12 | 1pmol | 0.494 | 2.19 |
| | | | 0mol | 0.226* | |
| 18 | 20 | 14 | 1pmol | 0.605 | 2.63 |
| | | | 0mol | 0.230* | |
| 19 | 20 | 16 | 1pmol | 0.503 | 2.57 |
| | | | 0mol | 0.196* | |
| 20 | 20 | 18 | 1pmol | 0.491 | 2.63 |
| | | | 0mol | 0.187* | |

| | | | | | |
|---|---|---|---|---|---|
| * Background value | | | | | |

**[Table 12]**

| Table 12. Background value and S/N value in measurement of modified nucleobase by absorbent polynucleotides of various lengths | | | | | |
|---|---|---|---|---|---|
| Absorbent polynucleotide | Length | Number of cytosines | Target nucleic acid (DNA) | OD450 | S/N |
| - | - | - | 1pmol | 0.656 | 2.16 |
| | | | 0mol | 0.304* | |
| 17 | 20 | 12 | 1pmol | 0.579 | 2.46 |
| | | | 0mol | 0.236* | |
| 21 | 30 | 12 | 1pmol | 0.526 | 2.13 |
| | | | 0mol | 0.247* | |
| 22 | 40 | 12 | 1pmol | 0.491 | 2.28 |
| | | | 0mol | 0.215* | |
| 23 | 50 | 12 | 1pmol | 0.430 | 2.69 |
| | | | 0mol | 0.160* | |

| | | | | | |
|---|---|---|---|---|---|
| * Background value | | | | | |

From the foregoing, it has been revealed that in the measurement of the modified nucleobase using the antibody against the modified nucleobase, use of the polynucleotide having 12 or more non-modified nucleobases as the absorbent polynucleotide produces an especially excellent effect.

### Example 5: Use of Different Capture Probe in Measurement of Modified Nucleobase Using Absorbent Polynucleotide

The nucleotide sequence of the target nucleic acid (DNA) is 5'-AATCAG[C]GGGAGCTCTTTCTTTGCGCGGCGTCCGTCCTGTTGACTTC-3' (SEQ ID NO: 29, [C] is 5-methylcytosine), and the nucleotide sequence of the capture probe for capturing the target nucleic acid is 5'-GAAGTCAACAGGACGACGCCGCGCAA-3' (SEQ ID NO: 2, the 5'-end is biotin-labeled); those artificially synthesized by Hokkaido System Science Co., Ltd. were used. The nucleotide sequence of the absorbent polynucleotide (DNA) containing cytosine is 5'-CCCCCCCCCCCCCCCCCCCC-3' (SEQ ID NO: 3, Absorbent Polynucleotide 1); that artificially synthesized by Hokkaido System Science Co., Ltd. was used. The capture probe was designed such that, when a hybrid with the target nucleic acid is formed, the modified nucleobase [C] was present in the single-stranded structure part of the hybrid.

The measurement of the modified nucleobase using the absorbent polynucleotide was carried out by a method similar to that in Example 1 except that a different target nucleic acid was used in different amounts (1 pmol, 0.1 pmol, or 0.01 pmol), that Absorbent Polynucleotide 1 was used in 5 pmol, and that 50 ng/mL of the anti-methylcytosine antibody (Clone33D3 manufactured by Nippon Gene Co., Ltd.) was used.

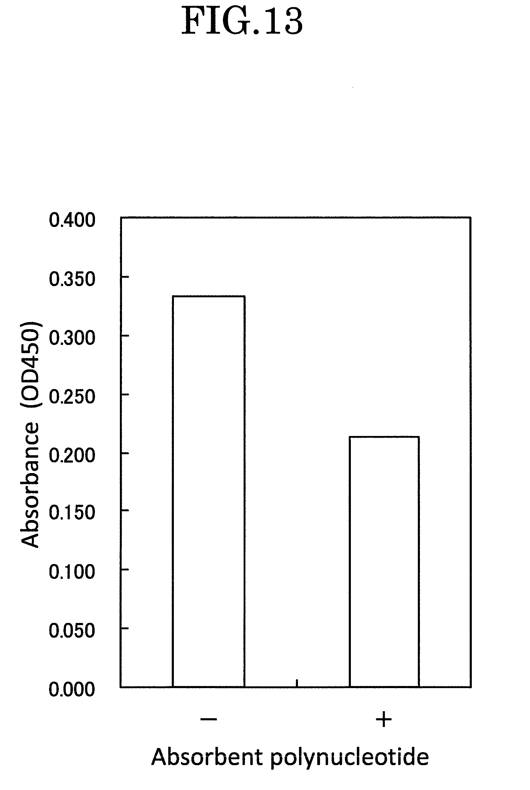

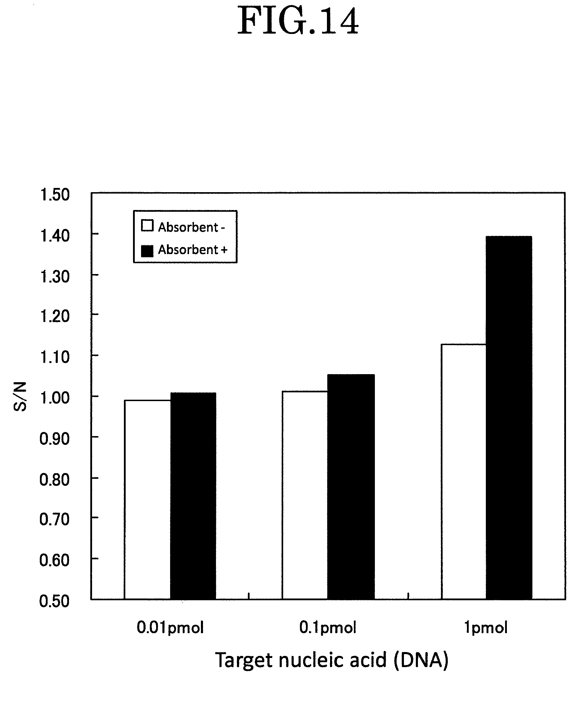

As a result of the measurement, when the hybrid of the target nucleic acid and the capture probe was formed, even when the capture probe was designed such that the modified nucleobase [C] was present in the single-stranded structure part of the hybrid, reductions in the background value and increases in the S/N value were revealed (Table 13 and FIGs. 13 and 14).

**[Table 13]**

| Table 13. Background value and S/N value in measurement of modified nucleobase by nucleic acid probe | | | |
|---|---|---|---|
| Absorbent polynucleotide | Target nucleic acid (DNA) | OD450 | S/N |
| - | 1pmol | 0.375 | 1.13 |
| | 0.1pmol | 0.336 | 1.01 |
| | 0.01pmol | 0.328 | 0.99 |
| | 0mol | 0.333* | |
| + | 1pmol | 0.295 | 1.39 |
| | 0.1pmol | 0.223 | 1.05 |
| | 0.01pmol | 0.214 | 1.01 |
| | 0mol | 0.212* | 1.13 |

| | | | |
|---|---|---|---|
| * Background value | | | |

From the foregoing, it has been revealed that when the target nucleic acid containing the modified nucleobase is measured by the antibody, not only the capture probe (Example 1) that was designed such that the unpaired part was formed at the modified nucleobase [C] in the double-stranded structure part of the hybrid, but also the capture probe (Example 5) that was designed such that the modified nucleobase [C] was present in the single-stranded part of the hybrid is effective.

### Example 6: Use of Different Target Nucleic Acid and Different Capture Probe in Measurement of Modified Nucleobase Using Absorbent Polynucleotide (1)

The nucleotide sequence of the target nucleic acid (DNA) is 5'-
G[C]GGAGCTCTCCCT[C]GGGA[C]GGTGGCAGCCTCGAGTGGTCCTGCA-3' (SEQ ID NO: 30, [C] is 5-methylcytosine), and the nucleotide sequence of the capture probe for capturing the target nucleic acid is 5'-TGCAGGACCACTCGAGGCTGCCAC-3' (SEQ ID NO: 31, the 5'-end is biotin-labeled); those artificially synthesized by Hokkaido System Science Co., Ltd. were used. The nucleotide sequence of the absorbent polynucleotide (DNA) containing cytosine is 5'-CCCCCCCCCCCCCCCCCCCC-3' (SEQ ID NO: 3, Absorbent Polynucleotide 1); that artificially synthesized by Hokkaido System Science Co., Ltd. was used. The capture probe was designed such that, when a hybrid with the target nucleic acid was formed, the modified nucleobase [C] was present in the single-stranded structure part of the hybrid.

The measurement of the modified nucleobase using the absorbent polynucleotide was carried out by a method similar to that in Example 1 except that a different target nucleic acid was used in different amounts (1 pmol, 0.1 pmol, or 0.01 pmol), that a different capture probe was used, that Absorbent Polynucleotide 1 was used in 25 pmol, and that 50 ng/mL of the anti-methylcytosine antibody (Clone33D3 manufactured by Nippon Gene Co., Ltd.) was used.

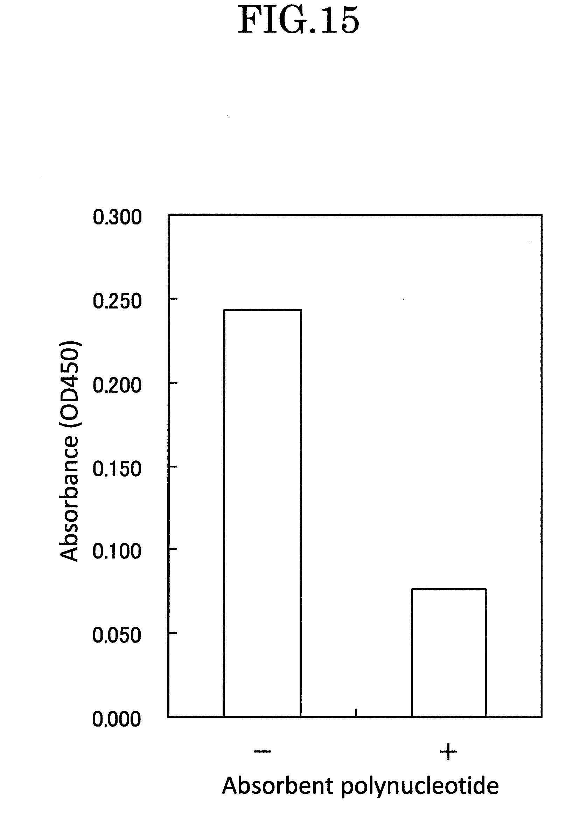

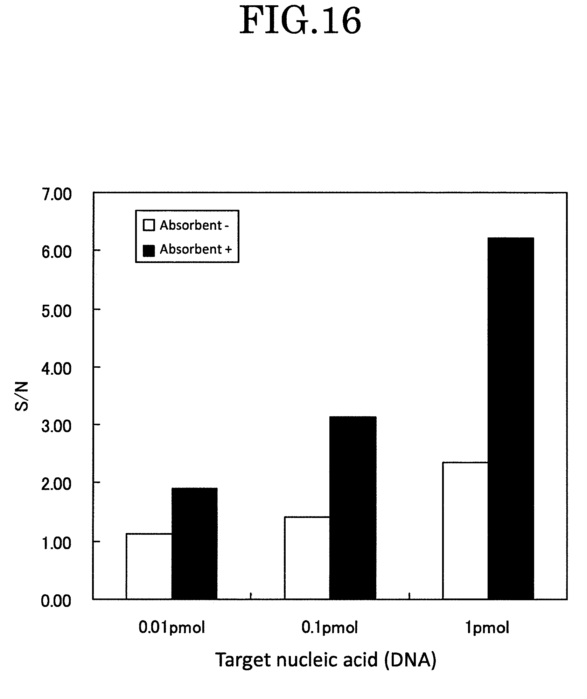

As a result of the measurement, in the measurement of the modified nucleobase using the absorbent polynucleotide, even when the target nucleic acid and the capture probe different from those of Example 1 and Example 5 were used, reductions in the background value and increases in the S/N value were revealed (Table 13 and FIGs. 15 and 16). When the target nucleic acid contained a plurality of modified nucleobases, even when the target nucleic acid was in an extremely small amount of 0.01 pmol (that is, even when the concentration of the target nucleic acid in the solution was set to 0.1 nM to cause hybridization with the capture probe and to form the hybrid), clear improvement of S/N (the absorbent polynucleotide: +) over S/N (the absorbent polynucleotide: -) was revealed. The experimental conditions on which the improvement was revealed in the present example were (the number of the modified nucleobases in the target nucleic acid = 3, and the amount of the target nucleic acid = 0.01 pmol). Meanwhile, the conditions on which the improvement was revealed in Example 5 were (the number of the modified nucleobases in the target nucleic acid = 1, and the amount of the target nucleic acid = 1 pmol). In other words, even though the number of the modified nucleobase of the present Example was only three times larger than the number of the modified nucleobase of Example 5, detection sensitivity about the amount of the target nucleic acid was increased by as much as an order of 10². As described above, when the target nucleic acid contained the modified nucleobases, detection sensitivity was amplified, whereby an especially excellent effect of the absorbent polynucleotide was revealed (Table 14 and FIG. 16).

**[Table 14]**

| Table 14. Background value and S/N value in measurement of modified nucleobase by nucleic acid probe | | | |
|---|---|---|---|
| Absorbent polynucleotide | Target nucleic acid (DNA) | OD450 | S/N |
| - | 1pmol | 0.571 | 2.35 |
| | 0.1pmol | 0.341 | 1.40 |
| | 0.01pmol | 0.272 | 1.12 |
| | 0mol | 0.243* | |
| + | 1pmol | 0.479 | 6.22 |
| | 0.1pmol | 0.241 | 3.14 |
| | 0.01pmol | 0.147 | 1.91 |
| | 0mol | 0.077* | |

| | | | |
|---|---|---|---|
| * Background value | | | |

### Example 7: Use of Different Target Nucleic Acid and Different Capture Probe in Measurement of Modified Nucleobase Using Absorbent Polynucleotide (2)

The nucleotide sequence of the target nucleic acid (DNA) is 5'-
G[C]GCAC[C]GTTTG[C]GACTTGGTGAGTGTCTGGGT[C]GCCT[C]GCTCC-3' (SEQ ID NO: 32, [C] is 5-methylcytosine), and the nucleotide sequence of the capture probe for capturing the target nucleic acid is 5'-ACCCAGACACTCACCAAGTC-3' (SEQ ID NO: 33, the 5'-end is biotin-labeled); those artificially synthesized by Hokkaido System Science Co., Ltd. were used. The nucleotide sequence of the absorbent polynucleotide (DNA) containing cytosine is 5'-CCCCCCCCCCCCCCCCCCCC-3' (SEQ ID NO: 3, Absorbent Polynucleotide 1); that artificially synthesized by Hokkaido System Science Co., Ltd. was used. The capture probe was designed such that, when a hybrid with the target nucleic acid was formed, the modified nucleobase [C] was present in the single-stranded structure part of the hybrid.

The measurement of the modified nucleobase using the absorbent polynucleotide was carried out by a method similar to that in Example 1 except that a different target nucleic acid was used in different amounts (1 pmol, 0.1 pmol, or 0.01 pmol), that a different capture probe was used, that Absorbent Polynucleotide 1 was used in 25 pmol, and that 50 ng/mL of the anti-methylcytosine antibody (Clone33D3 manufactured by Nippon Gene Co., Ltd.) was used.

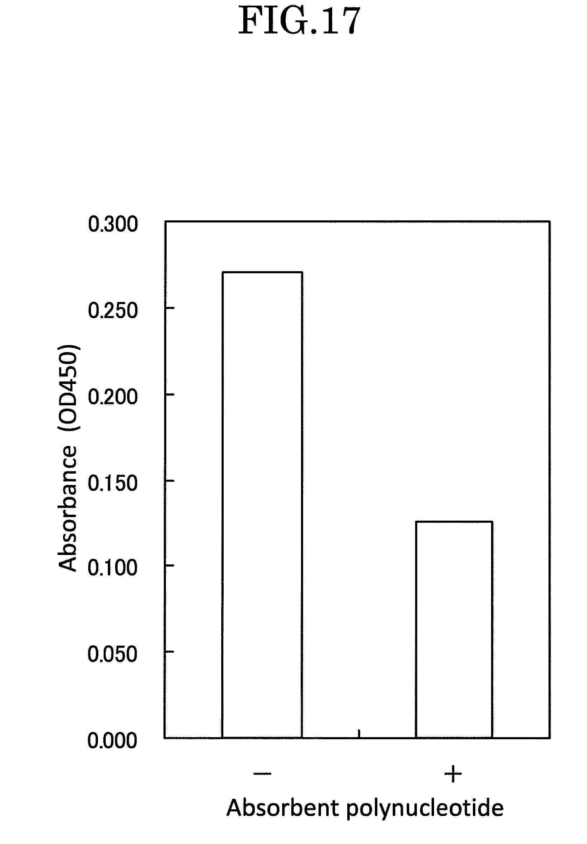

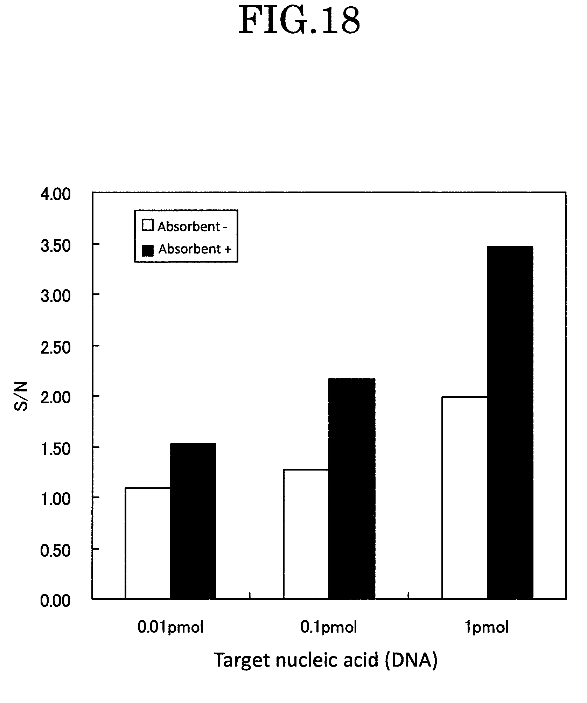

As a result of the measurement, in the measurement of the modified nucleobase using the absorbent polynucleotide, even when the target nucleic acid and the capture probe different from those of Example 1, Example 5, and Example 6 were used, reductions in the background value and increases in the S/N value were revealed (Table 14 and FIGs. 17 and 18). When the target nucleic acid contained a plurality of modified nucleobases, even when the target nucleic acid was in an extremely small amount of 0.01 pmol (that is, even when the concentration of the target nucleic acid in the solution was set to 0.1 nM to cause hybridization with the capture probe and to form the hybrid), clear improvement of S/N (the absorbent polynucleotide: +) over S/N (the absorbent polynucleotide: -) was revealed. The experimental conditions on which the improvement was revealed in the present example were (the number of the modified nucleobases in the target nucleic acid = 5, and the amount of the target nucleic acid = 0.01 pmol). Meanwhile, the conditions on which the improvement was revealed in Example 5 were (the number of the modified nucleobases in the target nucleic acid = 1, and the amount of the target nucleic acid = 1 pmol). In other words, even though the number of the modified nucleobases of the present Example is only five times larger than the number of the modified nucleobase of Example 5, detection sensitivity about the amount of the target nucleic acid was increased by as much as an order of 10². As described above, when the target nucleic acid contained the modified nucleobases, detection sensitivity was amplified, whereby an especially excellent effect of the absorbent polynucleotide was revealed (Table 15 and FIG. 18).

**[Table 15]**

| Table 15. Background value and S/N value in measurement of modified nucleobase by nucleic acid probe | | | |
|---|---|---|---|
| Absorbent polynucleotide | Target nucleic acid (DNA) | OD450 | S/N |
| - | 1pmol | 0.535 | 1.98 |
| | 0.1pmol | 0.343 | 1.27 |
| | 0.01pmol | 0.294 | 1.09 |
| | 0mol | 0.270* | |
| + | 1pmol | 0.435 | 3.46 |
| | 0.1pmol | 0.271 | 2.16 |
| | 0.01pmol | 0.192 | 1.53 |
| | 0mol | 0.126* | |

| | | | |
|---|---|---|---|
| * Background value | | | |

### Example 8: Use of Different Target Nucleic Acid and Different Capture Probe in Measurement of Modified Nucleobase Using Absorbent Polynucleotide (3)

The nucleotide sequence of the target nucleic acid (DNA) is 5'-GGCTCAGTTTACTAGTGCCATTTGTTCAGTGGTT[C]GT-3' (SEQ ID NO: 34, [C] is 5-methylcytosine), and the nucleotide sequence of the capture probe for capturing the target nucleic acid is 5'-
CACTGAACAAATGGCACTAGTAAACTGAGCCAAAAAAAAAA-3' (SEQ ID NO: 35, the 3'-end is biotin-labeled); those artificially synthesized by Hokkaido System Science Co., Ltd. were used. The nucleotide sequence of the absorbent polynucleotide (DNA) containing cytosine is 5'-
CCCCCCCCCCCCCCCCCCCCCCCCCCCCCCCCCCCCCCCC-3' (SEQ ID NO: 9, Absorbent Polynucleotide 4); that artificially synthesized by Hokkaido System Science Co., Ltd. was used. The capture probe was designed such that, when a hybrid with the target nucleic acid was formed, the modified nucleobase [C] was present in the single-stranded structure part of the hybrid.

The measurement of the modified nucleobase using the absorbent polynucleotide was carried out by a method similar to that in Example 1 except that a different target nucleic acid was used in different amounts (1 pmol, 0.1 pmol, or 0.01 pmol), that a different capture probe was used, that Absorbent Polynucleotide 4 was used in 25 pmol, and that 50 ng/mL of the anti-methylcytosine antibody (Clone33D3 manufactured by Nippon Gene Co., Ltd.) was used.

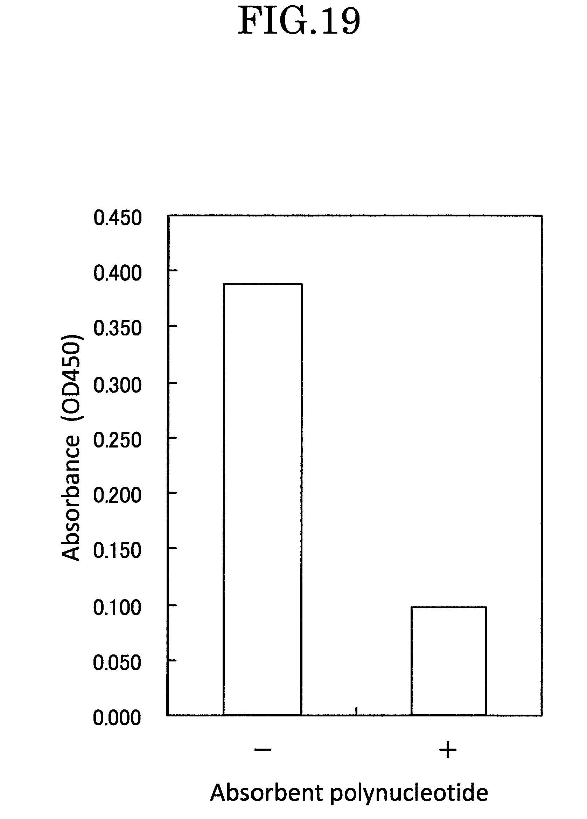

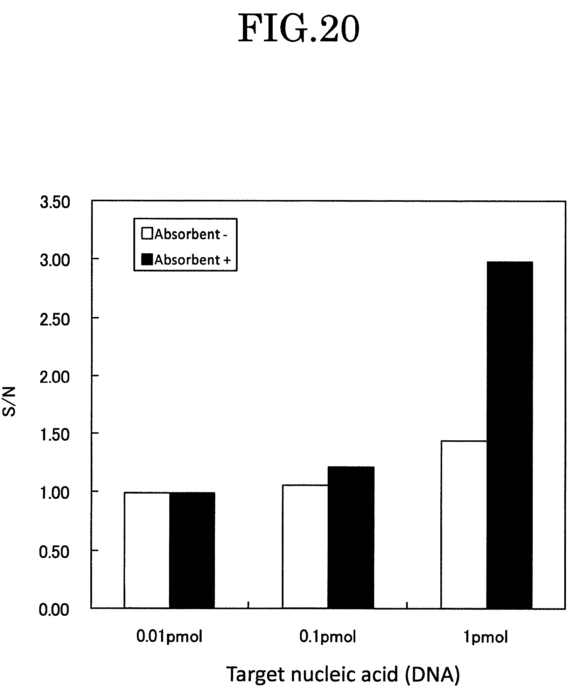

As a result of the measurement, in the measurement of the modified nucleobase using the absorbent polynucleotide, even when the target nucleic acid and the capture probe different from those of Example 1, Example 5, Example 6, and Example 7 were used, reductions in the background value and increases in the S/N value were revealed (Table 16 and FIGs. 19 and 20).

**[Table 16]**

| Table 16. Background value and S/N value in measurement of modified nucleobase by nucleic acid probe | | | |
|---|---|---|---|
| Absorbent polynucleotide | Target nucleic acid (DNA) | OD450 | S/N |
| - | 1pmol | 0.556 | 1.43 |
| | 0.1pmol | 0.409 | 1.05 |
| | 0.01pmol | 0.387 | 1.00 |
| | 0mol | 0.389* | |
| + | 1pmol | 0.289 | 2.97 |
| | 0.1pmol | 0.118 | 1.21 |
| | 0.1pmol | 0.097 | 1.00 |
| | 0mol | 0.097* | |

| | | | |
|---|---|---|---|
| * Background value | | | |

As demonstrated by Examples 1 and 5 to 8, it has been revealed that the present invention about the measurement of the modified nucleobase using the absorbent polynucleotide is useful for reducing the background value and increase the S/N value regardless of the types of the target nucleic acid and the capture probe.

### Industrial Applicability

The method and kit of the present invention are useful for measuring a modified nucleobase.

## Claims

1. A method for measuring a modified nucleobase
comprising:
(1) incubating a nucleic acid sample and a capture probe in a solution; and
(2) measuring the modified nucleobase using an antibody against the modified nucleobase in the presence of an absorbent polynucleotide in the solution obtained at (1).

2. The method according to claim 1, wherein
the nucleic acid sample contains a target nucleic acid containing a modified nucleobase, and the steps (1) and (2) are performed by (1') and (2'), respectively:
(1') reacting the nucleic acid sample containing the target nucleic acid containing the modified nucleobase and the capture probe in the solution by incubation to form a hybrid composed of the target nucleic acid and the capture probe; and
(2') measuring the modified nucleobase using the antibody in the presence of the absorbent polynucleotide in the solution containing the hybrid.

3. The method according to claim 1 or 2, wherein the target nucleic acid is a target nucleic acid potentially containing two or more modified nucleobases.

4. The method according to any one of claims 1 to 3, further comprising adding the capture probe to a solution containing the nucleic acid sample to prepare the solution containing both the nucleic acid sample and the capture probe.

5. The method according to any one of claims 1 to 4, wherein the nucleic acid sample is a sample containing a target DNA containing a modified nucleobase.

6. The method according to any one of claims 1 to 5, wherein the absorbent polynucleotide contains 12 or more nucleotide residues.

7. The method according to any one of claims 1 to 6, wherein the absorbent polynucleotide is a homopolynucleotide.

8. The method according to any one of claims 1 to 7, wherein the absorbent polynucleotide contains a nucleotide residue containing cytosine, and the modified nucleobase is a modified cytosine.

9. The method according to claim 8, wherein the modified cytosine is methylcytosine.

10. The method according to any one of claims 2 to 9, wherein the capture probe is designed such that an unpaired part of the modified nucleobase is formed in a doublestranded structure part of the hybrid.

11. The method according to any one of claims 2 to 10, wherein the capture probe is designed such that the modified nucleobase is present in a single-stranded structure part of the hybrid.

12. The method according to any one of claims 1 to 11, wherein the measurement of the modified nucleobase using the absorbent polynucleotide and the antibody is performed by ELISA.

13. A kit for measuring a modified nucleobase, the kit comprising:
(I) a capture probe;
(II) an absorbent polynucleotide; and
(III) an antibody against the modified nucleobase.


SEQUENCE LISTING

<110>  FUJIREBIO INC.

<120>  Methods of measuring modified nucleobases with absorbent
       polynucleotide and kits therefor

<130>  PRBA-26634

<150>  JP2013-171665
<151>  2013-08-21

<160>  35

<170>  PatentIn version 3.5

<210>  1
<211>  27
<212>  DNA
<213>  Artificial Sequence

<220>
<223>  Nucleotide sequence of a certain target nucleic acid.  The 13th
       nucleotide (cytosine) is methylated at position 5.

<400>  1
ttgcgcggcg tccgtcctgt tgacttc                                          27


<210>  2
<211>  26
<212>  DNA
<213>  Artificial Sequence

<220>
<223>  Nucleic acid probe for capturing the target nucleic acid of SEQ
       ID NO:1.

<400>  2
gaagtcaaca ggacgacgcc gcgcaa                                           26


<210>  3
<211>  20
<212>  DNA
<213>  Artificial Sequence

<220>
<223>  Poly C sequence (Absorbent polynucleotide 1)

<400>  3
cccccccccc cccccccccc                                                  20


<210>  4
<211>  20
<212>  DNA
<213>  Artificial Sequence

<220>
<223>  Poly T sequence (Control polynucleotide 1)

<400>  4

tttttttttt tttttttttt                                                20

<210>  5
<211>  20
<212>  DNA
<213>  Artificial Sequence

<220>
<223>  Poly A sequence (Control polynucleotide 2)

<400>  5
aaaaaaaaaa aaaaaaaaaa                                                20

<210>  6
<211>  20
<212>  DNA
<213>  Artificial Sequence

<220>
<223>  Poly G sequence (Control polynucleotide 3)

<400>  6
gggggggggg gggggggggg                                                20

<210>  7
<211>  10
<212>  DNA
<213>  Artificial Sequence

<220>
<223>  Poly C sequence (Absorbent polynucleotide 2)

<400>  7
cccccccccc                                                           10

<210>  8
<211>  30
<212>  DNA
<213>  Artificial Sequence

<220>
<223>  Poly C sequence (Absorbent polynucleotide 3)

<400>  8
cccccccccc cccccccccc cccccccccc                                     30

<210>  9
<211>  40
<212>  DNA
<213>  Artificial Sequence

<220>
<223>  Poly C sequence (Absorbent polynucleotide 4)

<400>  9
cccccccccc cccccccccc cccccccccc cccccccccc                          40

<210>  10
<211>  50
<212>  DNA
<213>  Artificial Sequence

<220>
<223>  Poly C sequence (Absorbent polynucleotide 5)

<400>  10
cccccccccc cccccccccc cccccccccc cccccccccc cccccccccc                50


<210>  11
<211>  60
<212>  DNA
<213>  Artificial Sequence

<220>
<223>  Poly C sequence (Absorbent polynucleotide 6)

<400>  11
cccccccccc cccccccccc cccccccccc cccccccccc cccccccccc cccccccccc     60


<210>  12
<211>  70
<212>  DNA
<213>  Artificial Sequence

<220>
<223>  Poly C sequence (Absorbent polynucleotide 7)

<400>  12
cccccccccc cccccccccc cccccccccc cccccccccc cccccccccc cccccccccc     60

cccccccccc                                                            70


<210>  13
<211>  12
<212>  DNA
<213>  Artificial Sequence

<220>
<223>  Poly C sequence (Absorbent polynucleotide 8)

<400>  13
cccccccccc cc                                                         12


<210>  14
<211>  14
<212>  DNA
<213>  Artificial Sequence

<220>
<223>  Poly C sequence (Absorbent polynucleotide 9)

<400>  14
cccccccccc cccc                                                       14


<210>  15

<211>  16
<212>  DNA
<213>  Artificial Sequence

<220>
<223>  Poly C sequence (Absorbent polynucleotide 10)

<400>  15
cccccccccc cccccc                                                        16


<210>  16
<211>  18
<212>  DNA
<213>  Artificial Sequence

<220>
<223>  Poly C sequence (Absorbent polynucleotide 11)

<400>  16
cccccccccc cccccccc                                                      18


<210>  17
<211>  20
<212>  DNA
<213>  Artificial Sequence

<220>
<223>  Absorbent polynucleotide 12

<400>  17
aaaaaacaaa aaacaaaaaa                                                    20


<210>  18
<211>  20
<212>  DNA
<213>  Artificial Sequence

<220>
<223>  Absorbent polynucleotide 13

<400>  18
aaacaaacaa acaaacaaaa                                                    20


<210>  19
<211>  20
<212>  DNA
<213>  Artificial Sequence

<220>
<223>  Absorbent polynucleotide 14

<400>  19
aacaacaaca acaacaacaa                                                    20


<210>  20
<211>  20
<212>  DNA
<213>  Artificial Sequence

<220>
<223>  Absorbent polynucleotide 15

<400>  20
aacacacaca acacacacaa                                                   20


<210>  21
<211>  20
<212>  DNA
<213>  Artificial Sequence

<220>
<223>  Absorbent polynucleotide 16

<400>  21
acacacacac acacacacac                                                   20


<210>  22
<211>  20
<212>  DNA
<213>  Artificial Sequence

<220>
<223>  Absorbent polynucleotide 17

<400>  22
ccacacacac cacacacacc                                                   20


<210>  23
<211>  20
<212>  DNA
<213>  Artificial Sequence

<220>
<223>  Absorbent polynucleotide 18

<400>  23
ccaccaccac caccaccacc                                                   20


<210>  24
<211>  20
<212>  DNA
<213>  Artificial Sequence

<220>
<223>  Absorbent polynucleotide 19

<400>  24
cccacccacc cacccacccc                                                   20


<210>  25
<211>  20
<212>  DNA
<213>  Artificial Sequence

<220>
<223>  Absorbent polynucleotide 20

<400>  25
   ccccccaccc cccacccccc                                                20

<210>  26
<211>  30
<212>  DNA
<213>  Artificial Sequence

<220>
<223>  Absorbent polynucleotide 21

<400>  26
ccacacacac cacacacaca aaaaaaaaac                                      30

<210>  27
<211>  40
<212>  DNA
<213>  Artificial Sequence

<220>
<223>  Absorbent polynucleotide 22

<400>  27
ccacacacac cacacacaca aaaaaaaaaa aaaaaaaaac                           40

<210>  28
<211>  50
<212>  DNA
<213>  Artificial Sequence

<220>
<223>  Absorbent polynucleotide 23

<400>  28
ccacacacac cacacacaca aaaaaaaaaa aaaaaaaaaa aaaaaaaaac                50

<210>  29
<211>  47
<212>  DNA
<213>  Artificial Sequence

<220>
<223>  Nucleotide sequence of a certain target nucleic acid.  The 7th
       nucleotide (cytosine) is methylated at position 5.

<400>  29
aatcagcggg agctctttct ttgcgcggcg tccgtcctgt tgacttc                   47

<210>  30
<211>  45
<212>  DNA
<213>  Artificial Sequence

<220>
<223>  Nucleotide sequence of a certain target nucleic acid.  The 2nd,
       15th and 20th nucleotides (cytosines) are methylated at position
       5.

<400>  30
gcggagctct ccctcgggac ggtggcagcc tcgagtggtc ctgca                45

<210>  31
<211>  24
<212>  DNA
<213>  Artificial Sequence

<220>
<223>  Nucleic acid probe for capturing the target nucleic acid of SEQ
       ID NO:30.

<400>  31
tgcaggacca ctcgaggctg ccac                                       24

<210>  32
<211>  44
<212>  DNA
<213>  Artificial Sequence

<220>
<223>  Nucleotide sequence of a certain target nucleic acid.  The 2nd,
       7th, 13th, 34th and 39 th nucleotides (cytosines) are methylated
       at position 5.

<400>  32
gcgcaccgtt tgcgacttgg tgagtgtctg ggtcgcctcg ctcc                 44

<210>  33
<211>  20
<212>  DNA
<213>  Artificial Sequence

<220>
<223>  Nucleic acid probe for capturing the target nucleic acid of SEQ
       ID NO:32.

<400>  33
acccagacac tcaccaagtc                                            20

<210>  34
<211>  37
<212>  DNA
<213>  Artificial Sequence

<220>
<223>  Nucleotide sequence of a certain target nucleic acid.  The 35th
       nucleotide (cytosine) is methylated at position 5.

<400>  34
ggctcagttt actagtgcca tttgttcagt ggttcgt                         37

<210>  35
<211>  41
<212>  DNA
<213>  Artificial Sequence

<220>
<223>  Nucleic acid probe for capturing the target nucleic acid of SEQ
      ID NO:34.

<400>  35
cactgaacaa atggcactag taaactgagc caaaaaaaaa a                          41